# EUROPEAN PATENT APPLICATION

(11) **EP 4 424 235 A1**
(43) Date of publication of application: **04.09.2024**
(21) Application number: 22886181.1
(22) Date of filing: 14.11.2022
(51) Int. Cl.: A61B 5/145, A61M 5/142

(54) **CLOSED-LOOP ARTIFICIAL PANCREATIC INSULIN INFUSION CONTROL SYSTEM**

(30) Priority: 25.10.2021 WO PCT/CN2021/126005; 24.10.2022 WO PCT/CN2022/127072
(71) Applicant: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2022/131685
(87) International publication number: WO 2023/072306

(57) **Abstract**

The invention discloses a closed-loop artificial pancreas insulin infusion control system, including: a detection module and an infusion module, a detection control unit is set in the detection module, an infusion control unit is set in the infusion module, and at least one calculation control unit is set in the detection module and the infusion module for calculating insulin infusion information and/or further processing of different insulin infusion information. Therefore, the closed-loop artificial pancreas insulin infusion control system can automatically switch the control unit according to different situations to avoid affecting the user experience due to the failure of a module or even bringing safety risks to the user, and to avoid replacing the whole device when only part of the function of some components fails, and to improve the utilization rate of each component of the closed-loop artificial pancreas.

## Description

### CROSS-REFERENCE TO RELATED APPLICATION

This application claims the priority benefit of PCT application no. PCT/CN2021/126005, filed on Oct 25, 2021 and PCT application no. PCT/CN2022/127072, filed on Oct 24, 2022.10.24. The entirety of the abovementioned patent application is hereby incorporated by reference herein and made a part of this specification.

### TECHNICAL FIELD

The present invention mainly relates to the field of medical device, and in particular, to a closed-loop artificial pancreas insulin infusion control system.

### BACKGROUND

The pancreas of healthy people can automatically secrete the required insulin/glucagon according to the glucose level in the human blood, thereby maintaining a reasonable range of blood glucose fluctuations. However, for diabetic patients, the function of their pancreas has been severely compromised, and the pancreas cannot secrete the required dosage of insulin. Therefore, diabetes mellitus is defined as a metabolic disease caused by abnormal pancreatic function, and it is also classified as one of the top three chronic conditions by the WHO. The present medical advancement has not been able to find a cure for diabetes mellitus. Yet, the best the technology could do is control the onset symptoms and complications by stabilizing the blood glucose level for diabetes patients.

Diabetic patients on an insulin pump need to check their blood glucose before infusing insulin into their bodies. At present, most detection methods can continuously detect blood glucose and send the blood glucose data to the remote device in real-time for the user to view. This detection method is called Continuous Glucose Monitoring (CGM), which requires the detection device to be attached to the surface of the patients' skin, and the sensor carried by the device to be inserted into the interstitial fluid for testing. According to the blood glucose (BG) level, the infusion system mimics an artificial pancreas to fill the gaps of the required insulin amount via the closed-loop pathway or the semi-closed-loop pathway.

At present, the control unit in the closed-loop artificial pancreatic insulin infusion control system is generally only set in one of the program modules of the infusion device or the program module of external electronic devices such as cell phones or handhelds or the program module of the detection device, once the device set with the control unit does not work properly, such as the location of external electronic devices is out of the normal range of use or malfunction, or when the calculation function in the infusion module or the detection module does not work, it will lead to the failure of the closed-loop artificial pancreatic insulin infusion control system. The user needs to replace the corresponding whole device in time to carry out normal drug infusion, reducing utilization of infusion modules or detection modules which affects the user experience, and if the replacement is not timely, it will lead to untimely drug infusion and bring safety risks to the user.

Therefore, in the prior art, there is an urgent need for a closed-loop artificial pancreas insulin infusion control system that can improve system component utilization and automatically switches control unit according to different situations.

### BRIEF SUMMARY OF THE INVENTION

The embodiment of the present invention discloses a closed-loop artificial pancreas insulin infusion control system. The system comprises a detection module and an infusion module, a detection control unit is set in the detection module, an infusion control unit is set in the infusion module, and at least one calculation control unit is set in the detection module and the infusion module for calculating insulin infusion information and/or further processing of different insulin infusion information. Therefore, the closed-loop artificial pancreas insulin infusion control system can automatically switch the control unit according to different situations to avoid affecting the user experience due to the failure of a module or even bringing safety risks to the user, and to avoid replacing the whole device when only part of the function of some components fails, and to improve the utilization rate of each component of the closed-loop artificial pancreas.

The invention discloses a closed-loop artificial pancreas insulin infusion control system, comprising: a detection module, provided with a detection control unit, used to control the detection module to detect blood glucose and communicate with external devices; and an infusion module, provided with an infusion control unit, used to control the infusion module to infuse currently needed insulin into a user's body and communicate with external devices; wherein, the infusion module is provided with a first calculation control unit and/or the detection module is provided with a second calculation control unit, the first calculation control unit and/or the second calculation control unit are preset with algorithms for calculating insulin infusion information based on the blood glucose value detected by the detection module through the preset algorithms and/or further processing different insulin infusion information.

According to one aspect of the present invention, at least one of the calculation control units is activated.

According to one aspect of the present invention, the insulin infusion information is determined by the detection module or the infusion module.

According to one aspect of the present invention, the determination method of the insulin infusion information is an independent determination method or a jointly determination method.

According to one aspect of the present invention, the independent determination method is that the detection module or the infusion module calculates the current insulin infusion information based on the blood glucose value provided by the detection module through the preset algorithms, respectively.

According to one aspect of the present invention, the independent determination method is that after the detection module or the infusion module calculates the current insulin infusion information based on the blood glucose value provided by the detection module through the preset algorithms, respectively, further processing the insulin infusion information and history information.

According to one aspect of the present invention, the jointly determination method is that the detection module and the infusion module perform calculation of the insulin infusion information, and the detection module or the infusion module perform the further processing to determine the insulin infusion information.

According to one aspect of the present invention, the jointly determination method is that one of the detection module and the infusion module calculates the insulin infusion information, and the other one further processes the insulin information and historical information after receiving the insulin information.

According to one aspect of the present invention, further includes an electronic module, provided with a third calculation control unit, which is preset with an algorithm for calculating the insulin infusion information based on the blood glucose value detected by the detection module through the preset algorithm and/or further processing different insulin infusion information.

According to one aspect of the present invention, one of the detection module, the infusion module or the electronic module determines the insulin infusion information.

According to one aspect of the present invention, the determination method of the insulin infusion information is an independent determination method or a jointly determination method.

According to one aspect of the present invention, the independent determination method is that the detection module, the infusion module or the electronic module calculates the current insulin infusion information based on the blood glucose value provided by the detection module through the preset algorithms, respectively.

According to one aspect of the present invention, the independent determination method is that after the detection module, the infusion module or the electronic module calculates the current insulin infusion information based on the blood glucose value provided by the detection module through the preset algorithms, respectively, further processing the insulin infusion information and history information.

According to one aspect of the present invention, the jointly determination method is that at least two modules of the electronic module, the detection module and the infusion module simultaneously calculate the insulin infusion information based on the blood glucose value provided by the detection module through their respective preset algorithms, respectively, and the module that ultimately determines the insulin infusion information performs further processing.

According to one aspect of the present invention, the module that ultimately determines the insulin infusion information itself also perform calculation of the insulin infusion information through the preset algorithm based on the blood glucose value provided by the detection module.

According to one aspect of the present invention, the module that ultimately determines the insulin infusion information itself does not perform calculation of the insulin infusion information.

According to one aspect of the present invention, the jointly determination method is that one of the detection module, the infusion module and the electronic module perform calculation of the insulin infusion information based on the blood glucose value detected by the detection module through the preset algorithm, and another module further processes the insulin information and historical information.

The invention discloses another closed-loop artificial pancreas insulin infusion control system, comprising: a detection module, provided with a detection control unit, used to control the detection module to detect blood glucose and communicate with external devices; and an infusion module, provided with an infusion control unit, used to control the infusion module to infuse currently needed insulin into a user's body and communicate with external devices; and an electronic module, communicating with the detection module and the infusion module; wherein, at least one of the infusion module, the detection module and the electronic module is provided with a calculation control unit, which is preset with an algorithm for calculating insulin infusion information based on the blood glucose value detected by the detection module through the preset algorithm and/or further processing different insulin infusion information.

According to one aspect of the present invention, at least one of the calculation control units is activated.

According to one aspect of the present invention, one of the infusion module, the detection module and the electronic module determines the insulin infusion information, the determination method of the insulin infusion information is an independent determination method or a jointly determination method.

According to one aspect of the present invention, the independent determination method is that the detection module, the infusion module or the electronic module calculates the current insulin infusion information based on the blood glucose value provided by the detection module through the preset algorithms, respectively.

According to one aspect of the present invention, the independent determination method is that after the detection module, the detection module or the electronic module calculates the current insulin infusion information based on the blood glucose value provided by the detection module through the preset algorithms, respectively, further processing the insulin infusion information and history information.

According to one aspect of the present invention, the jointly determination method is that at least two modules of the electronic module, the detection module and the infusion module simultaneously calculate the insulin infusion information based on the blood glucose value provided by the detection module through their respective preset algorithms, respectively, and the module that ultimately determines the insulin infusion information performs further processing.

According to one aspect of the present invention, the jointly determination method is that one of the detection module, the infusion module and the electronic module perform calculation of the insulin infusion information based on the blood glucose value detected by the detection module through the preset algorithm, and another module further processes the insulin information and historical information.

The invention discloses another closed-loop artificial pancreas insulin infusion control system, comprising: a detection module, provided with a detection control unit, used to control the detection module to detect blood glucose and communicate with external devices; and an infusion module, provided with an infusion control unit, used to control the infusion module to infuse currently needed insulin into a user's body and communicate with external devices; and an electronic module, communicating with the detection module and the infusion module; wherein, at least two of the infusion module, the detection module and the electronic module are provided with a calculation control unit, which is preset with an algorithm for calculating insulin infusion information based on the blood glucose value detected by the detection module through the preset algorithm and/or further processing different insulin infusion information; the module that determines the insulin infusion information is one of the modules provided with a calculation control unit, and can be switched according to different conditions.

According to one aspect of the present invention, at least two of the calculation control units are activated, and the module that determines the insulin infusion information is one of the modules that the calculation control unit is activated.

According to one aspect of the present invention, the preset algorithm is one of a classic PID algorithm, a classic MPC algorithm, a rMPC algorithm, a rPID algorithm or a compound artificial pancreas algorithm, and the rMPC algorithm or rPID algorithm is an algorithm that converts blood glucose that is asymmetric in the original physical space to a blood glucose risk that is approximately symmetric in the risk space based on the classic PID algorithm and the classic MPC algorithm.

According to one aspect of the present invention, the conversion method of blood glucose risk space of the rMPC algorithm and the rPID algorithm includes one or more of a segmented weighting conversion, a relative value conversion, a blood glucose risk index conversion, and an improved control variability grid analysis conversion.

According to one aspect of the present invention, the blood glucose risk conversion method of the rMPC algorithm and the rPID algorithm further include one or more of the following processing methods:
① subtract a component which is proportional to the predicted plasma insulin concentration;
② deduct the amount of insulin that has not yet worked in the body;
③ the autoregressive method is used to compensate for the detecting delay of interstitial fluid glucose concentration and blood glucose concentration.

According to one aspect of the present invention, the compound artificial pancreas algorithm, including a first algorithm and a second algorithm. The first algorithm is used to calculate the first insulin infusion amount I₁, the second algorithm is used to calculate the second insulin infusion volume I₂, and the compound artificial pancreas algorithm further optimizes I₁ and I₂ to obtain the final insulin infusion amount I₃.

According to one aspect of the present invention, the final insulin infusion amount I₃ is optimized by the average value I̅ of the first insulin infusion amount I₁ and the second insulin infusion amount I₂:
① obtain the average value I̅ of the first insulin infusion amount I₁ and the second insulin infusion amount I₂, and $\overline{I} = \frac{{I}_{1} + {I}_{2}}{2}$*;*
② substitute the average value *I̅̅̅* into the first algorithm and the second algorithm to adjust the algorithm parameters;
③ recalculate the first insulin infusion amount I₁ and the second insulin infusion amount I₂ based on the current blood glucose level and the first algorithm and the second algorithm with adjusted the parameters;
④ calculate steps ①∼③ cyclically until I₁=I₂, and the final insulin infusion amount I₃=I₁=I₂.

According to one aspect of the present invention, the final insulin infusion amount I₃ is optimized by the weighted value *I̅*' of the first insulin infusion amount I₁ and the second insulin infusion amount I₂:
① obtain the weighted value *I̅̅̅*' of the first insulin infusion amount I₁ and the second insulin infusion amount I₂, and *̅I̅*̅'̅ *= α* _{*} *I*₁ + *β* _{*} *I₂*, where a and β are the weighting coefficients of the first insulin infusion amount I₁ and the second insulin infusion amount I₂, respectively.
② substitute the average value *I̅'̅* into the first algorithm and the second algorithm to adjust the algorithm parameters;
③ recalculate the first insulin infusion amount I₁ and the second insulin infusion amount I₂ based on the current blood glucose level and the first algorithm and the second algorithm with adjusted the parameters;
④ calculate steps ①∼③ cyclically until I₁=I₂, and the final insulin infusion amount I₃=I₁=I₂.

According to one aspect of the present invention, the final insulin infusion amount I₃ is optimized by comparing the first insulin infusion amount I₁ and the second insulin infusion amount I₂ with the current statistical analysis result I₄: ${I}_{3} = { \begin{matrix}{I}_{1} , & \left|{I}_{1}-{I}_{4}\right| \leq \left|{I}_{2}-{I}_{4}\right| \\ {I}_{2} , & \left|{I}_{1}-{I}_{4}\right| > \left|{I}_{2}-{I}_{4}\right|\end{matrix} .$

Compared with the prior art, the technical solution of the present invention has the following advantages:
In the closed-loop artificial pancreas insulin infusion control system disclosed in the present invention, the detection control part and the calculation control part of the detection module are separated from one control unit, and the infusion control part and the calculation control part of the infusion module are separated. When one calculation control part in the system cannot work properly, it can be switched to another calculation control part in time, so as to avoid affecting the user experience due to the failure of one module of the control unit to work normally and even bringing security risks to the user, and to improve the utilization rate of each component of the closed-loop artificial pancreas.

Furthermore, the detection module and/or infusion module determine the current insulin infusion information in an independent determination method or a jointly determination method, making up for the lack of a properly working of the electronic module, further simplifying the closed-loop artificial pancreas system and reducing the user's cost of use.

Furthermore, the closed-loop artificial pancreatic insulin infusion control system also includes an electronic module, in which a calculation control unit can also be set. Therefore, the electronic module, infusion module, or detection module in the closed-loop artificial pancreatic insulin infusion control system can determine the insulin infusion information, and the determination method can be independent or joint determination method. In the joint determination method, the module that finally decides the insulin infusion information can perform calculation or not. The module that finally decides the insulin infusion information can also be switched according to different conditions to further enrich the way to determine the insulin infusion information to face more complex situations. In the joint determination method, one module can perform calculation, and the other module can perform further processing, which can share the calculation load of different calculation control units, average the power consumption of each module, and balance the use time of each module.

Furthermore, when a module calculates and determines the current insulin infusion information required by the preset algorithm independently, it can reduce the calculation load of other modules, reduce the power consumption of other modules, and extend their service life. In particular, since the detection module and/or infusion module are attached to the user's body, extending their service life can improve the user experience.

Furthermore, the current required insulin infusion information can be determined in a jointly method, which can further improve the accuracy of the user's required insulin infusion information.

Furthermore, the algorithm is one of the rMPC algorithm or the rPID algorithm, which converts the asymmetric blood glucose in the original physical space to the approximately symmetric blood glucose risk in the risk space, making full use of the advantages of rPID algorithm and rMPC algorithm to face complex scenarios, so that the artificial pancreas can provide reliable insulin infusion under various conditions. Blood glucose can reach the ideal level at the expected time, realising precise control for a closed-loop artificial pancreas insulin infusion system.

Furthermore, the final output of the compound artificial pancreas algorithm is the same result calculated by the rPID algorithm and rMPC algorithm, making the result more feasible and reliable.

Furthermore, the final output of the compound artificial pancreas algorithm is the same result obtained by averaging or weighting the different results calculated by the rPID algorithm and rMPC algorithm. The two sets of algorithms compensate each other to improve the accuracy of the output results.

Furthermore, the final output of the compound artificial pancreas algorithm is obtained by comparing the different results calculated by the rPID algorithm and rMPC algorithm, with the statistical analysis results of the historical data so as to ensure the reliability of the insulin infusion from another aspect.

### BRIEF DESCRIPTION OF THE DRAWINGS

FIG.1 is a schematic diagram of the module relationship of the closed-loop artificial pancreas insulin infusion control system according to one embodiment of the present invention.
FIG.2 is a comparison diagram of the blood glucose in the original physical space and the risk space, which is obtained through the segmented weighting and the relative value conversion according to an embodiment of the present invention.
FIG.3 is a comparison diagram of the blood glucose in the original physical space and the risk space, which is obtained through the BGRI and CVGA method according to an embodiment of the present invention.
FIG.4 is a schematic diagram of an insulin IOB curve according to an embodiment of the present invention.
FIG.5 is a schematic diagram of four types of mainstream clinical optimal basal rate settings according to an embodiment of the present invention
FIG.6 is a schematic diagram of the module relationship of the closed-loop artificial pancreas insulin infusion control system according to another embodiment of the present invention.
FIG.7 is a schematic diagram of the module relationship of the closed-loop artificial pancreas insulin infusion control system according to another embodiment of the present invention.
FIG.8 is a schematic diagram of the module relationship of the closed-loop artificial pancreas insulin infusion control system according to another embodiment of the present invention.
FIG.9a and FIG.9b are flow diagrams of a closed-loop artificial pancreas insulin infusion control system that determines insulin infusion information based on different priority conditions according to two other embodiments of the present invention.
FIG. 10 is a schematic diagram of the module relationship of the closed-loop artificial pancreas insulin infusion control system according to another embodiment of the present invention.
FIG.11 is a schematic diagram of the module relationship of the closed-loop artificial pancreas insulin infusion control system according to another embodiment of the present invention.

### DETAILED DESCRIPTION

As mentioned above, when the module set with the control unit does not work properly, it will lead to the failure of the closed-loop artificial pancreatic insulin infusion control system. The user needs to replace the corresponding device in time to carry out normal drug infusion, which affects the user experience, and if the replacement is not timely, it will lead to untimely drug infusion and bring safety risks to the user. At the same time, since the detection control part and the calculation control part of the detection module are not separated, and the infusion control part and the calculation control part of the infusion module are not separated, once the control unit fails, the whole detection module or the whole infusion module needs to be replaced, reducing the utilization rate of the detection module or the infusion module.

In order to solve this problem, the present invention provides a closed-loop artificial pancreas insulin infusion control system, the system comprises: a detection module and an infusion module, a detection control unit is set in the detection module, an infusion control unit is set in the infusion module, the infusion module is provided with a first calculation control unit and/or the detection module is provided with a second calculation control unit, the first calculation control unit and/or the second calculation control unit are preset with algorithms for calculating insulin infusion information based on the blood glucose value detected by the detection module through the preset algorithms and/or further processing different insulin infusion information. Therefore, the closed-loop artificial pancreas insulin infusion control system can automatically switch the control unit according to different situations to avoid affecting the user experience due to the failure of a module or even bringing safety risks to the user, and to avoid replacing the whole device when only part of the function of some components fails, and to improve the utilization rate of each component of the closed-loop artificial pancreas.

Various exemplary embodiments of the present invention will now be described in detail with reference to the drawings. The relative arrangement of the components and the steps, numerical expressions and numerical values set forth in the embodiments are not to be construed as limiting the scope of the invention.

In addition, it should be understood that, for ease of description, the dimensions of the various components shown in the figures are not necessarily drawn in the actual scale relationship, for example, the thickness, width, length or distance of certain units may be exaggerated relative to other parts.

The following description of the exemplary embodiments is merely illustrative, and is not intended to be in any way limiting the invention and its application or use. The techniques, methods, and devices that are known to those of ordinary skill in the art may not be discussed in detail, but such techniques, methods, and devices should be considered as part of the specification.

It should be noted that similar reference numerals and letters indicate similar items in the following figures. Therefore, once an item is defined or illustrated in a drawing, it will not be discussed further in the following description of the drawings.

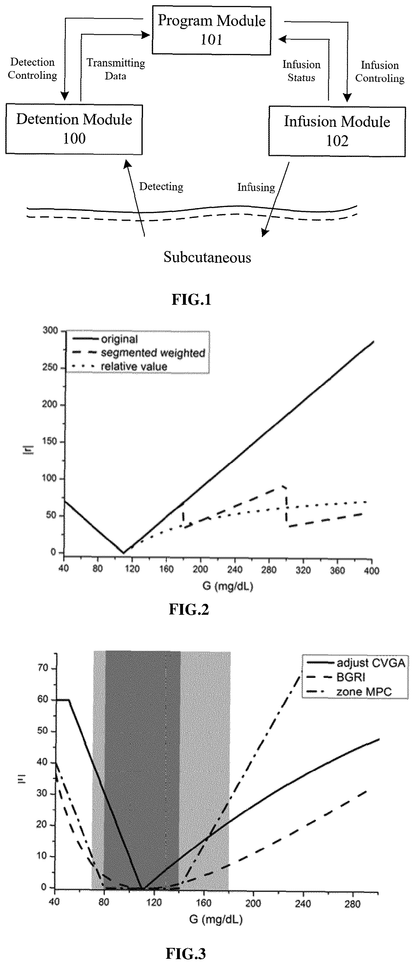

FIG.1 is a schematic diagram of the module relationship of the closed-loop artificial pancreas insulin infusion control system according to the embodiment of the present invention.

The closed-loop artificial pancreas insulin infusion control system disclosed in the embodiment of the present invention mainly includes a detection module 100, a program module 101, and an infusion module 102.

The detection module 100 is used to continuously detect the user's real-time blood glucose (BG) level. Generally, detection module 100 is a Continuous Glucose Monitoring (CGM) for detecting real-time BG, monitoring BG changes, and sending them to the program module 101.

Program module 101 is used to control the detection module 100 and the infusion module 102. Therefore, program module 101 is connected to detection module 100 and infusion module 102, respectively. Here, the connection refers to a conventional electrical connection or a wireless connection.

The infusion module 102 includes the essential mechanical assemblies used to infuse insulin and is controlled by program module 101. According to the current insulin infusion dose calculated by program module 101, infusion module 102 injects the current insulin dose required into the user's body. At the same time, the real-time infusion status of infusion module 102 can also be fed back to program module 101.

The embodiment of the present invention does not limit the specific positions and connection relationships of the detection module 100, the program module 101 and the infusion module 102, as long as the aforementioned functional conditions can be satisfied.

As in an embodiment of the present invention, the three are electrically connected to form a single part. Therefore, the three modules can be attached on only one position of the user's skin. If the three modules are connected as a whole and attached in only one position, the number of the device on the user skin will be reduced, thereby reducing the interference of more attached devices on user activities. At the same time, it also effectively solves the problem of poor wireless communication between separating devices, further enhancing the user experience.

Another embodiment of the present invention is that the program module 101 and the infusion module 102 are electrically connected to form a single part, while the detection module 100 is separately provided in another part. At this time, the detection module 100 and the program module 101 transmit wireless signals to realise the mutual connection. Therefore, program module 101 and infusion module 102 can be attached to the user's skin position while the detection module 100 is attached to the other position.

Another embodiment of the present invention is that the program module 101 and the detection module 100 are electrically connected, forming a single part, while the infusion module 102 is separately provided in another part. The infusion module 102 and the program module 101 transmit wireless signals to realise the mutual connection. Therefore, program module 101 and the detection module 100 can be attached to the same position of the user's skin while the infusion module 102 is attached to the other position.

Another embodiment of the present invention is that the three are provided in different parts, thus being attached to different positions. Simultaneously, program module 101, detection module 100, and infusion module 102 transmit wireless signals to realize the mutual connection.

It should be noted that the program module 101 of the embodiment of the present invention also has functions such as storage, recording, and access to the database. Thus, program module 101 can be reused. In this way, the user's physical condition data can be stored, but the production and consumption costs can be saved. As described above, when the service life of the detection module 100 or the infusion module 102 expires, program module 101 can be separated from the detection module 100, the infusion module 102, or both the detection module 100 and the infusion module 102.

Generally, the service lives of the detection module 100, the program module 101, and the infusion module 102 are different. Therefore, when the three are electrically connected to form a single device, the three can also be separated in pairs. For example, if one module expires, the user can only replace this module and keep the other two modules continuously using.

Here, it should be noted that the program module 101 of the embodiment of the present invention may also include multiple sub-modules. According to the functions of the sub-modules, different sub-modules can be respectively assembled in a different part, which is not a specific limitation herein, as long as the control conditions of the program module 101 can be satisfied.

Specifically, the program module 101 is preset with an rPID (risk-proportional-integral-derivative) algorithm that converts the asymmetric blood glucose in the original physical space to the approximately symmetric blood glucose in the risk space. The rPID algorithm is obtained by converting the classic PID (proportional-integral-derivative) algorithm. The specific converting method will be detailed below. According to the corresponding infusion instructions calculated by the rPID algorithm, module 101 controls the infusion Module 102 infuses insulin.

The classic PID algorithm can be expressed by the following formula: $PID \left(t\right) = C + {K}_{P} \left(G-{G}_{B}\right) + {K}_{I} \int \left(G-{G}_{B}\right) dt + {K}_{D} \frac{dG}{dt}$ Where:
K_{P} is the gain coefficient of the proportional part;
K_{I} is the gain coefficient of the integral part;
K_{D} is the gain coefficient of the differential part;
G represents the current blood glucose level;
G_{B} represents the target blood glucose level;
C represents a constant;
PID(t) represents the infusion instruction sent to the insulin infusion system.

Considering the actual distribution characteristics of glucose concentration in diabetic patients, for example, the normal blood glucose range is 80-140 mg/dL, and it can also be widened to 70-180 mg/dL. General hypoglycemia can reach 20-40 mg/dL, while high blood glucose can reach 400-600 mg/dL.

The distribution of high/low blood glucose (original physical space) has significant asymmetry. In clinical practice, the risk of high blood glucose and low blood glucose corresponding to the same degree of blood glucose deviation from the normal range will be significantly different, such as a decrease of 70 mg/dL, from 120mg/dL to 50mg/dL will be considered severe hypoglycemia, with high clinical risk, and emergency measures such as supplementing carbohydrates need to be taken. The increase of 70 mg/dL, from 120mg/dL to 190mg/dL is just beyond the normal range. For diabetic patients, the degree of high blood glucose is not serious, and it is often reached in daily situations, and there is no need to take treatment measures.

Considering the asymmetric characteristics of the clinical risk of glucose concentration, the asymmetric blood glucose in the original physical space is converted to the approximately symmetric blood glucose in risk space, making the PID algorithm more robust.

Correspondingly, the rPID algorithm formula is converted into the following form: $rPID \left(t\right) = C + {K}_{P} r + {K}_{I} \int rdt + {K}_{D} \frac{dr}{dt}$ Where:
rPID(t) represents the infusion instruction sent to the insulin infusion system after risk conversion;
r means blood glucose risk;
The meanings of other symbols are the same as described above.

In order to maintain the integration stability of PID, combined with the physiological effect of insulin to lower blood glucose, in one embodiment of the present invention, input parameter of the PID, blood glucose deviation amount Ge=G-GB is processed, such as segmented weighting (example: GB=110mg/dL), as follows: ${ \begin{matrix}r = Ge , if {G}_{B} < G \leq 180 mg / dL \\ r = Ge ∗ 0.5 , if 180 < G \leq 300 mg / dL \\ r = Ge ∗ 0.2 , if 300 < G \leq 400 mg / dL \\ r = \left(400-{G}_{B}\right) ∗ 0.2 , if G > 400 mg / dL\end{matrix}$

In another embodiment of the present invention, a blood glucose value greater than the target blood glucose G_{B} is converted by the relative value, as follows: ${ \begin{matrix}r = G - {G}_{B} = Ge , if G \leq {G}_{B} \\ r = 100 ∗ \frac{G - {G}_{B}}{G} = 100 ∗ \frac{Ge}{G} , if G > {G}_{B}\end{matrix}$

Fig. 2 is a comparison diagram of the blood glucose in the original physical space and the risk space obtained through the segmented weighting and the relative value conversion according to an embodiment of the present invention.

In the original PID algorithm, the blood glucose risk (ie Ge) on both sides of the target blood glucose value presents a severe asymmetry consisting of the original physical space. After being converted to the blood glucose risk space, the blood glucose risk on both sides of the target blood glucose value is approximately symmetric. In this way, the integral term can be kept stable, making the rPID algorithm more robust.

In another embodiment of the present invention, there is a fixed zero-risk point during risk conversion, and the data on both sides of the deviation from the zero-risk point is processed. The original parameter corresponding to greater than zero risk point is positive when converted to the risk space, and the original parameter corresponding to less than zero risk point is negative when converted to the risk space. Specifically, the classic blood glucose risk index (BGRI) method can be used. This method is based on clinical practice. It is believed that the clinical risks of 20mg/dL for hypoglycemia and 600mg/dL for hyperglycemia are equivalent. Through logarithm conversion, the overall blood glucose in the range of 20-600mg/dL is processed. The blood glucose concentration at zero risk point in this method is set as G_{B}. The risk space conversion formula is as follows: ${ \begin{matrix}r = - r \left(G\right) , if G \leq {G}_{B} ; \\ r = r \left(G\right) , if G > {G}_{B} ;\end{matrix}$ where: $r \left(G\right) = 10 ∗ f {\left(G\right)}^{2}$

The conversion function f(G) is as follows: $f \left(G\right) = 1.509 ∗ \left[{\left(ln\left(G\right)\right)}^{1.084}-5.381\right]$

In the classic blood glucose risk index (BGRI) method, the blood glucose concentration at zero risk point is 112mg/dL. In other embodiments of the present invention, the blood glucose concentration at the zero-risk point can also be adjusted in conjunction with clinical practice risks and data trends; there is no specific limitation here. When fitting the risk space of the blood glucose concentration where the blood glucose concentration is greater than that at zero risk point, the specific fitting method is not specifically limited.

In another embodiment of the present invention, an improved Control Variability Grid Analysis (CVGA) method is used. The blood glucose concentration at zero risk point is defined as 110 mg/dL in the original CVGA, and the following equal-risk blood glucose concentration data pairs are assumed (90 mg/dL, 180mg/dL; 70mg/dL, 300mg/dL; 50mg/dL, 400mg/dL). In the embodiment of the present invention, considering the real risks of clinical practice and the trend characteristics of the data, it was adjusted, and the risk data of (70mg/dL, 300mg/dL) was revised to (70mg/dL, 250mg/dL), and blood glucose concentration at zero risk point is defined as G_{B}. At the same time, a polynomial model is fitted to it, and the following risk functions for the two sides of the zero-risk point are obtained: ${ \begin{matrix}r = G - {G}_{B} , if G \leq {G}_{B} ; \\ r = - 4.8265 ∗ {10}^{4} - 4 ∗ {G}^{2} + 0.45563 ∗ G - 44.855 , if G > {G}_{B}\end{matrix}$

And the maximum value is limited as: $\left|r\right| = min \left(\left|r\right|, n\right)$

Where the range of the limit of the maximum value n is from 0 to 80mg/dL, preferably, the value of n is 60mg/dL.

In other embodiments of the present invention, the blood glucose concentration at the zero-risk point and equal risk data pairs can also be adjusted in conjunction with clinical practice risks and data trends, and there is no specific limitation here. When fitting equal risk data pairs, the specific fitting method is not specifically limited. The data used to limit the maximum is also not specifically limited here.

Fig. 3 is a comparison diagram of the blood glucose in the original physical space and the risk space, which has been obtained through the BGRI and CVGA method according to an embodiment of the present invention.

Similar to the treatment of Zone-MPC, within the normal range of blood glucose, the blood glucose risk after conversion by BGRI and CVGA methods is quite flat, especially within 80-140mg/dL. Unlike Zone-MPC, where the blood glucose risk is completely zero in this range, it loses the ability to adjust further. Although the blood glucose risk in rPID is smooth within this range, it still has a stable and slow adjustment ability, making blood glucose further adjust to close the target value to achieve more precise blood glucose control.

In another embodiment of the present invention, a unified processing method can be used for data deviating from both sides of the zero-risk point. As in the preceding embodiment, the BGRI or CVGA method can deal with the data deviating from both sides of the zero-risk point; Different treatment methods can also be used, such as combining the BGRI and CVGA methods at the same time. The glucose concentration at zero risk point blood is the same, such as G_{B}. When the blood glucose concentration is less than G_{B}, the BGRI method is used, and the blood glucose concentration is greater than G_{B}, the CVGA method is used. At this time: $r = - r \left(G\right) , if G \leq {G}_{B}$ where: $r \left(G\right) = 10 ∗ f {\left(G\right)}^{2}$

The conversion function f(G) is as follows: $f \left(G\right) = 1.509 ∗ \left[{\left(ln\left(G\right)\right)}^{1.084}-5.381\right]$ $r = - 4.8265 ∗ {10}^{4} - 4 ∗ {G}^{2} + 0.45563 ∗ G - 44.855 , if G > {G}_{B}$

Similarly, when the blood glucose concentration is great than G_{B}, the BGRI method is used, and the blood glucose concentration is less than G_{B}, the CVGA method is used. At this time: $r = r \left(G\right) , if G > {G}_{B}$ where: $r \left(G\right) = 10 ∗ f {\left(G\right)}^{2}$

The conversion function f(G) is as follows: $f \left(G\right) = 1.509 ∗ \left[{\left(ln\left(G\right)\right)}^{1.084}-5.381\right]$ $r = G - {G}_{B} , if G > {G}_{B}$

And the maximum value is limited as: $\left|r\right| = min \left(\left|r\right|, n\right)$

Where the range of the limit of the maximum value n is from 0 to 80mg/dL, preferably, the value of n is 60mg/dL.

In other embodiments of the present invention, the blood glucose level at the zero risk point can also be set as the target blood glucose value G_{B}, when the blood glucose concentration is less than G_{B}, the BGRI method is used, when the blood glucose concentration is great than G_{B}, such as segmented weighting or relative value converting.

When it is converted by segmented weighting, the formula is: $r = - r \left(G\right) , if G \leq {G}_{B}$ where: $r \left(G\right) = 10 ∗ f {\left(G\right)}^{2}$

The conversion function f(G) is as follows: $f \left(G\right) = 1.509 ∗ \left[{\left(ln\left(G\right)\right)}^{1.084}-5.381\right]$ ${ \begin{matrix}r = Ge , if {G}_{B} < G < 180 mg / dL \\ r = Ge ∗ 0 .5 , if 180 < G \leq 300 mg / dL \\ r = Ge ∗ 0 .2 , if 300 < G \leq 400 mg / dL \\ r = \left(400-{G}_{B}\right) ∗ 0 .2 , if G > 4 00 mg / dL\end{matrix}$

When it is converted by a relative value, the formula is: $r = - r \left(G\right) , if G \leq {G}_{B}$ where: $r \left(G\right) = 10 ∗ f {\left(G\right)}^{2}$

The conversion function f(G) is as follows: $f \left(G\right) = 1.509 ∗ \left[{\left(ln\left(G\right)\right)}^{1.084}-5.381\right]$ $r = 100 ∗ \frac{G - {G}_{B}}{G} = 100 ∗ \frac{Ge}{G} , if G > {G}_{B}$

When the blood glucose value at the zero risk point is the target blood glucose value G_{B}, for the data less than to the target blood glucose value G_{B}, when the segmented weighting converting, relative value converting, and CVGA method are used, the functions are the same. Therefore, when the blood glucose concentration is great than G_{B}, the BGRI method is used, when the blood glucose concentration is less than G_{B}, such as segmented weighting or relative value converting, the result is equivalent to the result that when the blood glucose value is less than the target blood glucose value G_{B}, the CVGA method is used when the blood glucose level is greater than the target blood glucose value G_{B}, the BGRI method is used, and the calculation formula is not repeated here.

In each embodiment of the present invention, the target blood glucose value G_{B} is 80-140 mg/dL; preferably, the target blood glucose value G_{B} is 110-120 mg/dL.

Through the above-converting methods, the asymmetric blood glucose in the original physical space can be converted to the approximately symmetric blood glucose in risk space in the rPID algorithm to retain the simplicity and robustness of the PID algorithm and control blood glucose risk with clinical value, to achieve precise control of the closed-loop artificial pancreatic insulin infusion system.

There are three major delay effects in the closed-loop artificial pancreas control system: insulin absorption delay (about 20 minutes from subcutaneous to blood circulation tissue, and about 100 minutes to liver), insulin onset delay (about 30-100 minutes), interstitial fluid glucose concentration and blood glucose detecting delay (approximately 5-15 minutes). Any attempt to accelerate the closed-loop responsiveness may result in unstable system behaviour and system oscillations. In order to compensate for the insulin absorption delay in the closed-loop artificial pancreas control system, in one embodiment of the present invention, an insulin feedback compensation mechanism is introduced. The amount of insulin that has not been absorbed in the body is subtracted from the output, which is a component that is proportional to the estimated plasma insulin concentration *γ* _{*} *Î_{P}*(*t*) (the plasma insulin concentration also regulates the actual human insulin secretion as a negative feedback Signal). The formula is as follows: ${PID}_{c} \left(t\right) = PID \left(t\right) - γ ∗ \hat{{I}_{P}} \left(t\right)$

Where:
PID(t) represents the infusion instruction sent to the insulin infusion system;
PIDc(t) represents the infusion instruction with compensation sent to the insulin infusion system;
γ represents the compensation coefficient of the estimated plasma insulin concentration to the algorithm output. If the coefficient increases, the algorithm will be relatively conservative, and if the coefficient decreases, the algorithm will be relatively aggressive. Therefore, in the embodiment of the present invention, the range of γ is 0.4-0.6. Preferably, γ is 0.5.

*Î_{P}*(*t*) represents the estimation of plasma insulin concentration, which various conventional prediction algorithms can obtain, for example, directly calculated from the infused insulin according to the pharmacokinetic curve of insulin, or using conventional autoregressive methods: $\hat{{I}_{P}} \left(n\right) = {K}_{0} ∗ {PID}_{c} \left(n-1\right) + {K}_{1} ∗ \hat{{I}_{P}} \left(n-1\right) + {K}_{2} ∗ \hat{{I}_{P}} \left(n-2\right)$

Where:
*Î_{P}*(*n*) represents the estimation of the plasma insulin concentration at the current moment;
*PID_{c}(n* - 1) represents the output with compensation at the previous moment;
*Î_{P}*(*n* - 1) represents the estimation of the plasma insulin concentration at the previous moment;
*Î_{P}*(*n* - 2) represents the estimation of the plasma insulin concentration at the time of up and up;
*K*₀ represents the coefficient of the output part with compensation at the previous moment;
*K*₁ represents the coefficient of the estimated part of the plasma insulin concentration at the previous moment;
*K*₂ represents the coefficient of the estimated part of the plasma insulin concentration at the previous time;
Where: *Îₚ*(*0*) = *K*_{0 *} *PID_{c}(0),* the time interval can be selected according to actual needs.

Correspondingly, the compensation output formula after risk conversion through the aforementioned method is as follows: ${rPID}_{c} \left(t\right) = rPID \left(t\right) - γ ∗ \hat{{I}_{P}} \left(t\right)$

Where:
*rPID_{c}(t)* represents the infusion instruction with compensation sent to the insulin infusion system after risk conversion;

The meanings of the other characters are as described above.

In order to compensate for the delay of insulin onset in the closed-loop artificial pancreas control system, in one embodiment of the present invention, insulin on board (IOB), which has not yet worked in the body, is introduced, and the IOB is subtracted from the output of insulin to prevent accumulation and overdose for insulin infusion, which can lead to risks such as postprandial hypoglycemia.

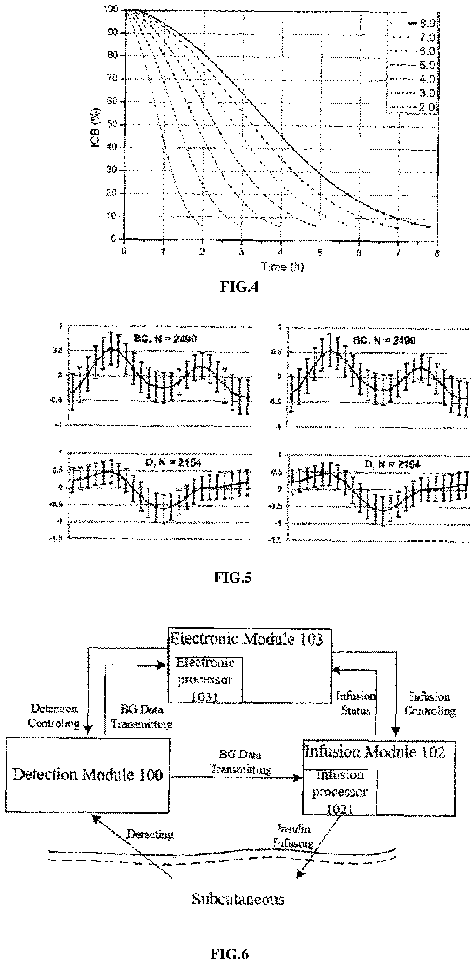

Fig. 4 is an insulin IOB curve according to an embodiment of the present invention.

According to the IOB curve shown in FIG. 4, the cumulative residual amount of insulin previously infused can be calculated, and the selection of the specific curve can be determined based on the actual insulin action time of the user. $PID ′ \left(t\right) = PID \left(t\right) - IOB \left(t\right)$

Where:
PID'(t) represents the infusion instruction sent to the insulin infusion system after deducting IOB;
PID(t) represents the infusion instruction sent to the insulin infusion system;
IOB(t) represents the amount of insulin that has not yet worked in the body at time t.

Correspondingly, the output formula after deducting the amount of insulin that has not yet worked in the body after risk conversion through the aforementioned method is as follows: $rPID ′ \left(t\right) = rPID \left(t\right) - IOB \left(t\right)$

Where:
*rPID'*(*t*) represents the infusion instruction sent to the insulin infusion system after risk conversion, deducting the amount of insulin that has not yet worked in the body;

The meanings of the other characters are as described above.

In order to obtain an ideal control effect, IOB(t) is divided into meal insulin IOBm and non-meal insulin IOBo. The formula is as follows: $IOB \left(t\right) = {IOB}_{m , t} + {IOB}_{o , t}$

Where: ${ \begin{matrix}{IOB}_{m , t} = {D}_{2} {I}_{m , t} \\ \begin{matrix}{IOB}_{o , t} = {D}_{2} {I}_{o , t} & if G > 300 mg / dL\end{matrix} \\ \begin{matrix}{IOB}_{o , t} = {D}_{4} {I}_{o , t} & if 300 mg / dL \geq G > 200 mg / dL\end{matrix} \\ \begin{matrix}{IOB}_{o , t} = {D}_{6} {I}_{o , t} & if 2 00 mg / dL \geq G > 140 mg / dL\end{matrix} \\ \begin{matrix}{IOB}_{o , t} = {D}_{8} {I}_{o , t} & if G \leq 140 mg / d L\end{matrix}\end{matrix}$

Where:
*IOB_{m,t}* represents the amount of meal insulin that has not yet worked in the body at time t;
*IOB_{o,t}* represents the amount of non-meal insulin that has not yet worked in the body at time t;
Di(i=2-8) represents the respective coefficients corresponding to the IOB curve with insulin action time i;
*I*_{*m*,*t*} represents the amount of meal insulin;
*I*_{*o*,*t*} represents the amount of non-meal insulin;
IOB(t) represents the amount of insulin that has not yet worked in the body at time t.

Dividing the IOB into meal and non-meal insulin can make insulin cleared faster when meals ingesting or blood sugar are too high and can obtain greater insulin output and regulate blood glucose more quickly. When approaching the target, a longer insulin action time curve is used to make insulin clear more slowly, and blood sugar regulation is more conservative and stable.

When PID'(t)>0 or rPID'(t)>0, the final insulin infusion amount is PID'(t) or rPID'(t);

When PID'(t)<0 or rPID'(t)<0, the final insulin infusion amount is 0.

In an embodiment of the present invention, an autoregressive method is used to compensate for detecting delay of interstitial fluid glucose concentration and blood glucose concentration. The formula is as follows: ${G}_{SC} \left(n\right) = {K}_{0} ∗ \hat{G} \left(n-1\right) + {K}_{1} ∗ {G}_{SC} \left(n-1\right) + {K}_{2} ∗ {G}_{SC} \left(n-2\right)$

Where:
*G_{SC}*(*n*) represents the glucose concentration in the interstitial fluid at the current moment, that is, the measured value of the detecting system;
$\hat{{G}_{P}} \left(n-1\right)$ represents the estimated concentration of blood glucose at the previous moment;
*G_{SC}*(*n* - 1) and *G_{SC}*(*n* - 2) represent the glucose concentration in the interstitial fluid at the first previous time and the second previous time, respectively;
*K*₀ represents the coefficient of the estimated concentration of blood glucose at the previous moment;
*K*₀₁ and *K*₂ respectively represent the coefficient of glucose concentration in the interstitial fluid at the first previous time and the second previous time, respectively.

Where: *Ĝ*(0) = *G_{SC}*(*0*)

The blood glucose concentration is estimated by the interstitial fluid glucose concentration, which compensates for the detecting delay of the interstitial fluid glucose concentration and blood glucose, making the PID algorithm more accurate. Correspondingly, the rPID algorithm can also more accurately calculate the actual insulin demand for the human body.

In the embodiment of the present invention, the insulin absorption delay, the insulin onset delay, the detecting delay of interstitial fluid glucose concentration and blood glucose can be partially compensated or fully compensated. Preferably, all delay factors are considered fully compensated for making the rPID algorithm more accurate.

In another embodiment of the present invention, the program module 101 is preset with an rMPC (risk-model-predict-control) algorithm that converts the asymmetric blood glucose in the original physical space to the approximately symmetric blood glucose in the risk space. The rMPC algorithm is obtained by converting the classic MPC (risk-model-predict-control) algorithm. According to the corresponding infusion instructions calculated by the rMPC algorithm, program module 101 controls infusion Module 102 infuses insulin.

The classic MPC algorithm consists of three elements, the prediction model, the value function and the constraints. The classic MPC prediction model is as follows: ${x}_{t + 1} = {Ax}_{t} + {BI}_{t}$ ${G}_{t} = {Cx}_{t}$

Where:
*x*_{*t*+1} represents the state parameter at the next moment, ${x}_{t + 1} = \left[\begin{matrix}{G}_{t + 1} \\ {G}_{t} \\ {G}_{t - 1}\end{matrix}\right]$;
*xₜ* represents the current state parameter, ${x}_{t} = \left[\begin{matrix}G \\ {G}_{t - 1} \\ {G}_{t - 2}\end{matrix}\right]$;
*Iₜ* represents the amount of insulin infusion at the current moment;
*Gₜ* represents the blood glucose concentration at the current moment.

The parameter matrix is as follows: $A = \left[\begin{matrix}{b}_{1} & {b}_{2} & {b}_{3} \\ 1 & 0 & 0 \\ 0 & 1 & 0\end{matrix}\right]$ $B = \left[\begin{matrix}{k}_{i} \\ 0 \\ 0\end{matrix}\right]$ $C = \left[\begin{matrix}1 & 0 & 0\end{matrix}\right]$

Where:
b1, b2, b3, Ki are initial values.

The value function of the MPC algorithm is composed of the sum of squared deviations of the output G (blood glucose level) and the sum of squared changes of the input I (insulin amount). The MPC algorithm needs to obtain the minimum solution of the value function. ${J}^{MPC} = {\sum }_{j = 1}^{p} {‖ {G}_{t + j}^{e} ‖}^{2} + R {\sum }_{j = 1}^{N} {‖ {I}_{t + j}^{′} ‖}^{2}$

Where:
${I}_{t + j}^{′}$ represents the change of insulin infusion after step j;
${G}_{t + j}^{e}$ represents the difference between the predicted blood glucose concentration and the target blood glucose value after step j;
t represents the current moment;
N and P are the number of steps in the control time window and the predictive time window, respectively;
R is the weighting coefficient of the insulin component.

The amount of insulin infusion at step j is*Iₜ* + *I_{t+j.}*

In the embodiment of the present invention, the control time window Tc=30min, the prediction time window Tp=60min, and the weighting coefficient R of the amount of insulin is 11000. It should be noted that although the control time window used in the calculation is 30min, only the first step calculation result of insulin output is used in the actual operation. After the operation, the minimum solution of the above value function is recalculated according to the latest blood glucose data obtained.

In the embodiment of the present invention, the infusion time step in the control time window is jₙ, and the range of jₙ is 0-30 min, preferably 2 min. The number of steps N=T_{c}/jₙ, and the range of j is 0 to N.

In other embodiments of the present invention, the weighting coefficients of the amount of insulin, the control time window and the predicted time window can also be selected as other values, which are not specifically limited here.

As mentioned above, the distribution of high/low blood glucose (original physical space) has significant asymmetry. The risk of high blood glucose and low blood glucose corresponding to the same degree of blood glucose deviation from the normal range will be significantly different in clinical practice. Considering the asymmetric characteristics of the clinical risk of glucose concentration, the asymmetric blood glucose in the original physical space is converted to the approximately symmetric blood glucose in risk space, making the MPC algorithm more accurate and flexible.

The value function of the rMPC algorithm after risk conversion is as follows: ${J}^{rMPC} = {\sum }_{j = 1}^{p} {‖ {r}_{t + j} ‖}^{2} + R {\sum }_{j = 1}^{N} {‖ {I}_{t + j}^{′} ‖}^{2}$

Where:
*rₜ₊ⱼ* represents the blood glucose risk index after step j;
${I}_{t + j}^{′}$ represents the change of insulin infusion after step j.

The deviation of blood glucose value is converted to the corresponding blood glucose risk. The specific conversion method is the same as that in the aforementioned rPID algorithm, such as segmented weighting and relative value converting; it also includes setting a fixed zero risk point in the risk space. The blood glucose concentration at the zero risk point can be set as the target blood glucose value. Data on both sides deviating from the zero risk point are processed, such as using BGRI and the improved CVGA method; it also includes different methods for processing data that deviates from the target blood glucose value.

Specifically, when the segmented weighting converting is used: ${ \begin{matrix}{r}_{t + j} = \left({G}_{t + j}-{G}_{B}\right) , if {G}_{B} < {G}_{t + j} \leq 180 mg / dL \\ {r}_{t + j} = \left({G}_{t + j}-{G}_{B}\right) * 0.5 , if 180 < {G}_{t + j} \leq 300 mg / dL \\ {r}_{t + j} = \left({G}_{t + j}-{G}_{B}\right) ∗ 0.2 , if 300 < {G}_{t + j} \leq 400 mg / dL \\ {r}_{t + j} = \left(400-{G}_{B}\right) ∗ 0.2 , if {G}_{t + j} > 400 mg / dL\end{matrix}$

When the relative value converting is used: ${ \begin{matrix}{r}_{t + j} = {G}_{t + j} - {G}_{B} , if {G}_{t + j} \leq {G}_{B} \\ {r}_{t + j} = 100 ∗ \frac{{G}_{t + j} - {G}_{B}}{{G}_{t + j}} , if {G}_{t + j} > {G}_{B}\end{matrix}$

When the BGRI method is used: ${ \begin{matrix}{r}_{t + j} = - r \left({G}_{t + j}\right) , if {G}_{t + j} \leq {G}_{B} \\ {r}_{t + j} = r \left({G}_{t + j}\right) , if {G}_{t + j} > {G}_{B}\end{matrix}$

Where: $r \left({G}_{t + j}\right) = 10 ∗ f {\left({G}_{t + j}\right)}^{2}$

The conversion function f(Gₜ₊ⱼ) is as follows: $f \left({G}_{t + j}\right) = 1.509 ∗ \left[{\left(ln\left({G}_{t + j}\right)\right)}^{1.084}-5.381\right]$

When the CVGA method is used: ${ \begin{matrix}{r}_{t + j} = {G}_{t + j} - {G}_{B} , if {G}_{t + j} \leq {G}_{B} \\ {r}_{t + j} = - 4.8265 ∗ {10}^{4} - 4 ∗ {{G}_{t + j}}^{2} + 0.45563 ∗ {G}_{t + j} - 44.855 , if {G}_{t + j} > {G}_{B}\end{matrix}$

And the maximum value is limited as: $\left|{r}_{t + j}\right| = min \left(\left|{r}_{t + j}\right|, n\right)$

Where the range of the limit of the maximum value n is from 0 to 80mg/dL, preferably, the value of n is 60mg/dL.

If the detected blood glucose concentration in step j *Gₜ₊ⱼ* is less than G_{B}, the BGRI method will be used. If the detected blood glucose concentration in step j *Gₜ₊ⱼ* is greater than G_{B}, the CVGA method will be used: ${r}_{t + j} = - r \left({G}_{t + j}\right) , if {G}_{t + j} \leq {G}_{B}$

Where: $r \left({G}_{t + j}\right) = 10 ∗ f {\left({G}_{t + j}\right)}^{2}$

The conversion function f(Gₜ₊ⱼ) is as follows: $f \left({G}_{t + j}\right) = 1.509 ∗ \left[{\left(ln\left({G}_{t + j}\right)\right)}^{1.084}-5.381\right]$ ${r}_{t + j} = - 4.8265 ∗ {10}^{4} - 4 ∗ {{G}_{t + j}}^{2} + 0.45563 ∗ {G}_{t + j} - 44.855 , if {G}_{t + j} > {G}_{B}$

If the detected blood glucose concentration in step j *Gₜ₊ⱼ* is great than G_{B}, the BGRI method will be used. If the detected blood glucose concentration in step j *G*_{*t*+*j*} is less than G_{B}, the CVGA method will be used: ${r}_{t + j} = r \left({G}_{t + j}\right) , if {G}_{t + j} > {G}_{B}$

Where: $r \left({G}_{t + j}\right) = 10 ∗ f {\left({G}_{t + j}\right)}^{2}$

The conversion function f(Gₜ₊ⱼ) is as follows: $f \left({G}_{t + j}\right) = 1.509 ∗ \left[{\left(ln\left({G}_{t + j}\right)\right)}^{1.084}-5.381\right]$ ${{r}_{t}}_{+ j} = {G}_{t + j} - {G}_{B} , if {G}_{t + j} \leq {G}_{B}$

And the maximum value is limited as: $\left|r\right| = min \left(\left|r\right|, n\right)$

Where the range of the limit of the maximum value n is from 0 to 80mg/dL, preferably, the value of n is 60mg/dL.

If the detected blood glucose concentration in step j *Gₜ₊ⱼ* is less than G_{B}, the BGRI method will be used. If the detected blood glucose concentration in step j *Gₜ₊ⱼ* is great than G_{B}, the segmented weighting converting will be used: ${r}_{t + j} = - r \left({G}_{t + j}\right) , if {G}_{t + j} \leq {G}_{B}$

Where: $r \left({G}_{t + j}\right) = 10 ∗ f {\left({G}_{t + j}\right)}^{2}$

The conversion function f(Gₜ₊ⱼ) is as follows: $f \left({G}_{t + j}\right) = 1.509 ∗ \left[{\left(ln\left({G}_{t + j}\right)\right)}^{1.084}-5.381\right]$ ${ \begin{matrix}{r}_{t + j} = \left({G}_{t + j}-{G}_{B}\right) , if {G}_{B} < {G}_{t + j} \leq 180 mg / dL \\ {r}_{t + j} = \left({G}_{t + j}-{G}_{B}\right) ∗ 0.5 , if 180 < {G}_{t + j} \leq 300 mg / dL \\ {r}_{t + j} = \left({G}_{t + j}-{G}_{B}\right) ∗ 0.2 , if 300 < {G}_{t + j} \leq 400 mg / dL \\ {r}_{t + j} = \left(400-{G}_{B}\right) ∗ 0.2 , if {G}_{t + j} > 400 mg / dL\end{matrix}$

When the detected blood glucose concentration in step j *Gₜ₊ⱼ* is less than G_{B}, the BGRI method is used, when the detected blood glucose concentration in step j *G*_{*t*+*j*} is great than G_{B}, the relative value converting is used: ${r}_{t + j} = - r \left({G}_{t + j}\right) , if {G}_{t + j} \leq {G}_{B}$

Where: $r \left({G}_{t + j}\right) = 10 ∗ f {\left({G}_{t + j}\right)}^{2}$

The conversion function f(Gₜ₊ⱼ) is as follows: $f \left({G}_{t + j}\right) = 1.509 ∗ \left[{\left(ln\left({G}_{t + j}\right)\right)}^{1.084}-5.381\right]$ ${r}_{t + j} = 100 ∗ \frac{{G}_{t + j} - {G}_{B}}{{G}_{t + j}} , if {G}_{t + j} > {G}_{B}$

For the data less than the target blood glucose value GB, the functions are the same when the segmented weighting converting, relative value converting, and CVGA method is used. Therefore, when the blood glucose concentration is great than G_{B}, the BGRI method is used, when the blood glucose concentration is less than G_{B}, such as segmented weighting or relative value converting, the result is equivalent to the result that when the blood glucose value is less than the target blood glucose value G_{B}, the CVGA method is used when the blood glucose level is greater than the target blood glucose value G_{B}, the BGRI method is used, and the calculation formula is not repeated here.

It should be noted that in the above conversion formulas:
*rₜ₊ⱼ* represents the blood glucose risk index at step j;
*Gₜ₊ⱼ* represents the blood glucose level detected in step j.

The target blood glucose value G_{B} is 80-140 mg/dL, preferably, the target blood glucose value G_{B} is 110-120 mg/dL.

The beneficial effects after risk conversion and the comparison of the relationship between blood glucose and blood glucose risk are consistent with the rPID algorithm and will not be repeated here.

Similarly, in order to compensate for the insulin absorption delay, the insulin feedback compensation mechanism can be used; in order to compensate for the delay of insulin onset, IOB can be used; in order to compensate for detecting delay of interstitial fluid glucose concentration and blood glucose concentration, the autoregressive method can be used. The specific compensation method is also consistent with the rPID algorithm, specifically:
For insulin absorption delay, the compensation formula is as follows: ${rI}_{c \left(t+j\right)} = {I}_{t + j} - γ \hat{{I}_{P}} \left(t+j\right)$

Where:
*Iₜ₊ⱼ* represents the infusion instruction sent to the insulin infusion system after step j;
*rI*_{*c (t*+*j)*} represents the infusion instruction with compensation sent to the insulin infusion system after step j;
γ represents the compensation coefficient of the estimated plasma insulin concentration to the algorithm output. If the coefficient increases, the algorithm will be relatively conservative, and if the coefficient decreases, the algorithm will be relatively aggressive. Therefore, in the embodiment of the present invention, the range of γ is 0.4-0.6. Preferably, γ is 0.5.
$\hat{{I}_{P}} \left(t+j\right)$ represents the estimation of plasma insulin concentration after step j.

For the delay of insulin onset, the compensation formula is as follows: ${rI}_{t + j}^{′} = {rI}_{t + j} - IOB \left(t+j\right)$

Where:
*rI*_{*t*+*j*} represents the infusion instruction sent to the insulin infusion system after deducting IOB at step j after risk conversion;
*rI*_{*t*+*j*} represents the infusion instruction sent to the insulin infusion system at step j after risk conversion;
*IOB*(*t* + *j*) represents the amount of insulin that has not yet worked in the body at time t+j.

Similarly, IOB(t+j) can be divided into meal insulin and non-meal insulin. The formula is as follows: $IOB \left(t+j\right) = {IOB}_{m , t + j} + {IOB}_{o , t + j}$

Where: ${ \begin{matrix}{IOB}_{m , t + j} = {D}_{2} {I}_{m , t + j} \\ {IOB}_{o , t + j} = {D}_{2} {I}_{o , t + j} if {G}_{t + j} > 300 mg / dL \\ {IOB}_{o , t + j} = {D}_{4} {I}_{o , t + j} if 300 mg / dL \geq {G}_{t + j} > 200 mg / dL \\ {IOB}_{o , t + j} = {D}_{6} {I}_{o , t + j} if 200 mg / dL \geq {G}_{t + j} > 140 mg / dL \\ {IOB}_{o , t + j} = {D}_{8} {I}_{o , t + J} if {G}_{t + j} \leq 140 mg / d L\end{matrix}$

Where:
*IOB*_{*m,t*+*j*} represents the amount of meal insulin that has not yet worked in the body at time t+j;
*IOB_{o,t}* + *j* represents the amount of non-meal insulin that has not yet worked in the body at time t+j;
Di(i=2-8) represents the respective coefficients corresponding to the IOB curve with insulin action time i;
*I*_{*m,t*+*j*} represents the amount of meal insulin at time t+j;
*I*_{*o,t*+*j*} represents the amount of non-meal insulin at time t+j;
IOB(t+j) represents the amount of insulin that has not yet worked in the body at time t+j.

When ${rI}_{t + j}^{′} > 0$, the final insulin infusion amount is ${rI}_{t + j}^{′}$;

When ${rI}_{t + j}^{′} < 0$, the final insulin infusion amount is 0.

The autoregressive method is used to detect the delay of interstitial fluid glucose concentration and blood glucose concentration.
the formula is as follows: ${G}_{SC} \left(t+j\right) = {K}_{0} ∗ \hat{G} \left(t+j-1\right) + {K}_{1} ∗ {G}_{SC} \left(t+j-1\right) + {K}_{2} ∗ {G}_{SC} \left(t+j-2\right)$

Where:
*G_{SC}*(*t* + j) represents the glucose concentration in the interstitial fluid at the time t+j, that is, the measured value of the detecting system;
$\hat{{G}_{P}} \left(t+j-1\right)$ represents the estimated concentration of blood glucose at the time t+j-1;
*G_{SC}*(*t* + *j* - 1) and *G_{SC}*(*t* + *j* - 2) represent the glucose concentration in the interstitial fluid at the time t+j-1 and t+j-2, respectively;
*K*₀ represents the coefficient of the estimated concentration of blood glucose at the time t+j-1;
*K*₀₁ and *K*₂ respectively represent the coefficient of glucose concentration in the interstitial fluid at the time t+j-1 and t+j-2, respectively.

Where: *Ĝ*(0) = *G_{SC}(0)*

The beneficial effects of various compensation methods are consistent with those in the rPID algorithm, which will not be repeated here.

In the rMPC algorithm, it is preferable to compensate for the delay of insulin onset and the detecting delay of interstitial fluid glucose concentration and blood glucose concentration.

In another embodiment of the present invention, the compound artificial pancreas algorithm is preset in program module 101. The compound artificial pancreas algorithm includes a first algorithm and a second algorithm. When the detection module 100 detects the current blood glucose level and sends the current blood glucose level to the program module 101, the first algorithm calculates the first insulin infusion amount I₁, the second algorithm calculates the second insulin infusion amount I₂, the compound artificial pancreas algorithm optimises the first insulin infusion amount I₁ and the second insulin infusion amount I₂ to obtain the final insulin infusion, and send the final insulin infusion amount I₃ to the infusion module 102, and the infusion module 102 performs insulin infusion according to the final infusion amount I₃.

The first and second algorithms are classic PID algorithms, the classic MPC algorithm, the rMPC algorithm, or the rPID algorithm. The rMPC algorithm or rPID algorithm is an algorithm that converts blood glucose that is asymmetric in the original physical space to a blood glucose risk that is approximately symmetric in the risk space. The conversion method of blood glucose risk in rMPC algorithm and rPID algorithm is as described above.

If I₁=I₂, then I₃=I₁=I₂;
If I₁≠I₂, then substitutes the average arithmetic value of I₁ and I₂ into the first and second algorithm to optimise the parameters, and then recalculate the current insulin infusion amount I₁ and I₂. If the data are not the same, repeat the above process until I₃=I₁=I₂, that is:
① obtain the average value *I̅* of the first insulin infusion amount I₁ and the second insulin infusion amount I₂, and $\overline{I} = \frac{{I}_{1} + {I}_{2}}{2}$;
② substitute the average value *I̅* into the first algorithm and the second algorithm to adjust the algorithm parameters;
③ recalculate the first insulin infusion amount I₁ and the second insulin infusion amount I₂ based on the current blood glucose level and the first algorithm and the second algorithm with adjusted the parameters;
④ calculate steps ①∼③ cyclically until I₁=I₂ and the final insulin infusion amount I₃=I₁=I₂.

At this time, when the first algorithm or the second algorithm is PID or rPID algorithm, the algorithm parameter is K_{P}, and K_{D} = T_{D} /K_{P}, T_{D} can be 60min-90 min, K_{I}=T_{I}*K_{P}, T_{I} can be 150min-450 min. When the first algorithm or the second algorithm is the MPC or rPMC algorithm, the algorithm parameter is K.

If I₁≠I₂, then the weighted value of I1 and I2 is substituted into the first and second algorithms to optimise the parameters and then recalculate the current insulin infusion amount I₁ and I₂. If the data are not the same, adjust the weighting coefficient to repeat the above process until I₃=I₁=I₂, that is:
① obtain the weighted value *I̅̅̅*'̅ of the first insulin infusion amount I₁ and the second insulin infusion amount I₂, and *I̅̅̅'̅* = *α* _{*} *I*₁ + *β* * *I*₂, where a and β are the weighting coefficients of the first insulin infusion amount I₁ and the second insulin infusion amount I₂, respectively.
② substitute the average value *̅I̅*̅'̅ into the first algorithm and the second algorithm to adjust the algorithm parameters;
③ recalculate the first insulin infusion amount I₁ and the second insulin infusion amount I₂ based on the current blood glucose level and the first algorithm and the second algorithm with adjusted the parameters;
④ calculate steps ①∼③ cyclically until I₁=I₂, and the final insulin infusion amount I₃=I₁=I₂.

Similarly, when the first algorithm or the second algorithm is PID or rPID algorithm, the algorithm parameter is K_{P}, and K_{D} = T_{D} /K_{P}, T_{D} can be 60min-90 min, K_{I}=T_{I}*K_{P}, T_{I} can be 150min-450 min. When the first algorithm or the second algorithm is the MPC or rPMC algorithm, the algorithm parameter is K.

In the embodiment of the present invention, α and β can be adjusted according to the first insulin infusion amount I₁ and the second insulin infusion amount I₂. When I₁≥I₂, α≤β; when I₁≤I₂, α≥β; preferably, α+β=1. In other embodiments of the present invention, α and β may also be other value ranges, which are not specifically limited here.

When the calculation results of the two are the same, that is, I₃=I₁=I₂, it can be considered that the amount of insulin infusion at the current moment can make the blood glucose level reach the ideal level. Through the processing mentioned above, the algorithms are mutually referenced. Preferably, the first algorithm and the second algorithm are the rMPC algorithm and the rPID algorithm, which are mutually referenced to improve the accuracy of the output further and make the result more feasible and reliable.

In another embodiment of the present invention, the program module 101 also provides a memory that stores the user's historical physical state, blood glucose level, insulin infusion, and other information. Statistical analysis can be performed based on the information in the memory to obtain the current statistical analysis result I₄, when I₁≠I₂, compare I₁, I₂ and I₄ to calculate the final insulin infusion amount I₃, the one that is closer to the statistical analysis result I₄ is selected as a result of the compound artificial pancreas algorithm, that is the final insulin infusion amount I₃, and the program module 101 sends the final insulin infusion amount I₃ to the infusion module 102 to infuse; ${I}_{3} = { \begin{matrix}{I}_{1} , & \left|{I}_{1}-{I}_{4}\right| \leq \left|{I}_{2}-{I}_{4}\right| \\ {I}_{2} , & \left|{I}_{1}-{I}_{4}\right| > \left|{I}_{2}-{I}_{4}\right|\end{matrix}$

Through comparison with historical data, the reliability of insulin infusion is ensured, on the other hand.

In another embodiment of the present invention, when I₁ and I₂ are inconsistent, and the difference is large, the blood glucose risk space conversion method in the rMPC algorithm and/or rPID algorithm and/or the compensation method regarding the delay effect can also be changed to adjust and make them more closely, and then finally determine the output result of the compound artificial pancreas algorithm through the above arithmetic average, weighting processing, or comparison with the statistical analysis result.

In another embodiment of the present invention, the closed-loop artificial pancreas control system further includes a meal recognition module and/or a motion recognition module, used to identify whether the user is eating or exercising. Commonly used meal identification can be determined based on the rate of blood glucose change and compared with a specific threshold. The rate of blood glucose change can be calculated from two moments or obtained by linear regression at multiple moments within a period of time. Specifically, when the rate of change at the two moments is used for calculation, the calculation formula is: $d {G}_{t} / dt = \left({G}_{t}-{G}_{t - 1}\right) / Δt$ where:
*Gₜ* represents the blood glucose level at the current moment;
*G*_{*t*-1} represents the blood glucose level at the previous moment;
Δt represents the time interval between the current moment and the last moment.

When the rate of change at three moments is used for calculation, the calculation formula is: $d {G}_{t} / dt = \left(3{G}_{t}-4{G}_{t - 1}+{G}_{t - 2}\right) / 2 Δt$ where:
*Gₜ* represents the blood glucose level at the current moment;
*G*_{*t*-1} represents the blood glucose level at the previous moment;
*G*_{*t*-2} represents the blood glucose level at the second previous moment
Δt represents the time interval between the current moment and the last moment.

Before calculating the blood glucose change rate, the original continuous glucose data can also be filtered or smoothed. The threshold can be set to 1.8mg/mL-3mg/mL or personalised.

Similar to meal recognition, exercise can cause a rapid drop in blood glucose. Therefore, exercise recognition can also be detected based on the rate of blood glucose change and a specific threshold. The rate of blood glucose change can also be calculated as described above, and the threshold can be personalised.

In order to determine the occurrence of movement more quickly, the closed-loop artificial pancreas insulin infusion control system further includes a movement sensor (not shown). The motion sensor automatically detects the user's physical activity, and the program module 101 can receive physical activity status information. The motion sensor can automatically and accurately sense the user's physical activity state and send the activity state parameters to the program module 101 to improve the output reliability of the compound artificial pancreas algorithm in exercise scenarios.

The motion sensor is provided in detection module 100, the program module 101 or the infusion module 102. Preferably, in the embodiment of the present invention, the motion sensor is provided in the program module 101.

It should be noted that the embodiment of the present invention does not limit the number of motion sensors and the installation positions of these multiple motion sensors, as long as the conditions for the motion sensor to sense the user's activity status can be satisfied.

The motion sensor includes a three-axis acceleration sensor or a gyroscope. The three-axis acceleration sensor or gyroscope can more accurately sense the body's activity intensity, activity mode or body posture. Preferably, in the embodiment of the present invention, the motion sensor combines a three-axis acceleration sensor and a gyroscope.

It should be noted that in the calculation process, the blood glucose risk conversion methods used by the rMPC algorithm and the rPID algorithm can be the same or different, and the compensation methods for the delay effect can also be the same or different. The calculation process can also be adjusted based on actual conditions.

FIG.6 is a schematic diagram of the module relationship of the closed-loop artificial pancreas insulin infusion control system according to another embodiment of the present invention.

In other embodiments of the present invention, the closed-loop artificial pancreas insulin infusion control system mainly includes a detection module 100, an infusion module 102, and an electronic module 103.

The detection module 100 is used to detect the user's real-time blood glucose level continuously. Generally, the detection module 100 is a continuous glucose monitor (Continuous Glucose Monitoring, CGM), which can detect blood glucose levels in real-time, monitor blood glucose changes, and send the current blood glucose levels to the infusion module 102 and the electronic module 103.

The infusion module 102 includes the mechanical assembly necessary for insulin infusion and other components capable of executing the first algorithm, such as an infusion processor 1021, controlled by the electronic module 103. The infusion module 102 receives the current blood glucose level sent by the detection module 100, calculates the first insulin infusion amount I₁ currently required through the first algorithm and sends the calculated first insulin infusion amount I₁ to the electronic module 103.

The electronic module 103 is used to control the operation of detection module 100 and the infusion module 102. Therefore, the electronic module 103 is connected to the detection module 100 and the infusion module 102, respectively. Here, the electronic module 103 is an external electronic device such as a mobile phone or a handset, and the connection refers to a wireless connection. The electronic module 103 includes a second processor. In the embodiment of the present invention, the second processor is capable of executing the second algorithm and the third algorithm, such as an electronic processor 1031. After the electronic module 103 receives the current blood sugar level, the current required second insulin infusion amount I₂ is calculated through the second algorithm. The first and second algorithms used by the electronic module 103 and the infusion module 102 to calculate the amount of insulin currently required are different.

After the electronic module 103 receives the first insulin infusion amount I₁ sent by the infusion module 102, it further optimises the first insulin infusion amount I₁ and the second insulin infusion amount I₂ through the third algorithm to obtain the final insulin infusion amount I₃, and sends final insulin infusion amount I₃ to the infusion module 102, the infusion module 102 injects the currently needed insulin amount I₃ into the user's body. At the same time, the infusion status of the infusion module 102 can also be fed back to the electronic module 103 in real-time. The specific optimisation method is as described above, which is:
If I₁=I₂, then I₃=I₁=I₂;

If I₁≠I₂, the electronic module 103 further substitutes the average arithmetic value of the two or the weighted value into the algorithm to recalculate the current insulin infusion amount I₁ and I₂. If the data are not the same, repeat the above process until I₃=I₁=I₂, that is:
① obtain the average value *I̅* of the first insulin infusion amount I₁ and the second insulin infusion amount I₂, and $\overline{I} = \frac{{I}_{1} + {I}_{2}}{2}$;
② substitute the average value *I̅* into the first algorithm and the second algorithm to adjust the algorithm parameters;
③ recalculate the first insulin infusion amount I₁ and the second insulin infusion amount I₂ based on the current blood glucose level and the first algorithm and the second algorithm with adjusted the parameters;
④ calculate steps ①∼③ cyclically until I₁=I₂, and the final insulin infusion amount I₃=I₁=I₂.

Or:
① obtain the average value *I̅*' of the first insulin infusion amount I₁ and the second insulin infusion amount I₂, and *I̅'* = *α* _{*} *I*₁ + *β* _{*} *I*₂, where a and β are the weighting coefficients of the first insulin infusion amount I₁ and the second insulin infusion amount I₂, respectively.
② substitute the average value *I̅'̅* into the first algorithm and the second algorithm to adjust the algorithm parameters;
③ recalculate the first insulin infusion amount I₁ and the second insulin infusion amount I₂ based on the current blood glucose level and the first algorithm and the second algorithm with adjusted the parameters;
④ calculate steps ①∼③ cyclically until I₁=I₂, and the final insulin infusion amount I₃=I₁=I₂.

When I₁≠I₂, the electronic module 103 can also compare I₁, hand I₄, which is a statistical analysis result at the current time by analysing the historical information based on the user's body state, blood sugar level and insulin infusion at each time in the past. The one that is closer to the statistical analysis result I₄ is selected as the final insulin infusion amount I₃, and the electronic module 103 sends the final insulin infusion amount I₃ to the infusion module 102 to infuse; ${I}_{3} = { \begin{matrix}{I}_{1} , & \left|{I}_{1}-{I}_{4}\right| \leq \left|{I}_{2}-{I}_{4}\right| \\ {I}_{2} , & \left|{I}_{1}-{I}_{4}\right| > \left|{I}_{2}-{I}_{4}\right|\end{matrix}$

In the embodiment of the present invention, the user's historical information may be stored in the electronic module 103 or a cloud management system (not shown), and the cloud management system and the electronic module 103 are connected wirelessly.

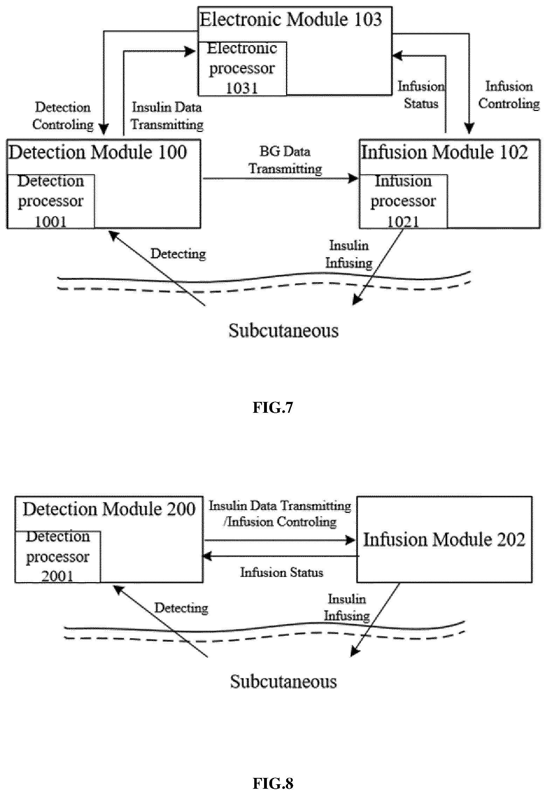

FIG.7 is a schematic diagram of the module relationship of the closed-loop artificial pancreas insulin infusion control system according to another embodiment of the present invention.

In the embodiments of the present invention, the closed-loop artificial pancreas insulin infusion control system mainly includes a detection module 100, an infusion module 102, and an electronic module 103.

The detection module 100 is used to detect the user's real-time blood glucose level continuously. Generally, the detection module 100 is a continuous glucose monitor (Continuous Glucose Monitoring, CGM), which can detect blood glucose levels in real-time, monitor blood glucose changes, and the current blood glucose levels have only been sent to the infusion module 102. The detection module 100 further includes a second processor. In the embodiment of the present invention, the second processor is capable of executing the second algorithm, such as a detection processor 1001. After detecting the real-time blood glucose level, detection module 100 directly calculates the second insulin infusion amount I₂ through the second algorithm and sends the calculated second insulin infusion amount I₂ to the electronic module 103.

As mentioned above, infusion module 102, as mentioned above, after receiving the current blood glucose level sent by the detection module 100, calculates the first insulin infusion amount I₁ currently required through the first algorithm and sends the calculated first insulin infusion amount I₁ to the electronic module 103. The first and second algorithms used by the electronic module 103 and the infusion module 102 to calculate the amount of insulin currently required are different.

After the electronic module 103 receives the first insulin infusion amount I₁ sent by the infusion module 102 and the second insulin infusion amount I₂ sent by the detection module 103, it further optimises the first insulin infusion amount I₁ and the second insulin infusion amount I₂ through the third algorithm to obtain the final insulin infusion amount I₃. It sends the final insulin infusion amount I₃ to the infusion module 102. The infusion module 102 injects the currently needed insulin amount I₃ into the user's body. At the same time, the infusion status of the infusion module 102 can also be fed back to the electronic module 103 in real-time. The specific optimisation method is as described above.

In the above two embodiments of the present invention, after the detection module 100 detects the current blood glucose level, the infusion processor 1021 preliminarily calculates the first insulin infusion amount I₁. The second processor (such as the electronic processor 1031 and the detection processor 1001) preliminarily calculate the second insulin infusion amount I₂, and I1 and I2 being sent to the electronic module 103. The electronic module 103 performs further optimisation and then sends the optimised final insulin infusion amount I₃ to the infusion module 102 to infuse insulin, improving the accuracy of infusion instructions.

In the above two embodiments of the present invention, the first algorithm and the second algorithm are one of the classic PID algorithms, the classic MPC algorithm, the rMPC algorithm, or the rPID algorithm. The advantages of using the rPID or rMPC algorithm to calculate are as described above, and the beneficial effects of other optimisation methods are also as described above and will not be repeated here.

The embodiment of the present invention does not limit the specific position and connection relationship of the detection module 100 and the infusion module 102, as long as the aforementioned functional conditions can be met.

As in an embodiment of the present invention, the two modules are electrically connected to form an integral assembly and are pasted in the same place on the user's skin. If the two modules are connected as a whole and pasted in the same position, the number of user skin pasting devices will be reduced, thereby reducing the interference of more pasted devices on user activities; at the same time, it also effectively solves the problem of poor wireless communication between separate devices, which further enhance the user experience.

As in another embodiment of the present invention, the two modules are arranged in different components and are passed on different positions of the user's skin. The detection module 100 and the infusion module 102 transmit wireless signals to realise the mutual connection.

FIG.8 is a schematic diagram of the module relationship of the closed-loop artificial pancreas insulin infusion control system according to another embodiment of the present invention.

The closed-loop artificial pancreas insulin infusion control system disclosed in the embodiment of the present invention mainly includes a detection module 200 and an infusion module 202. The detection module 100 is used to continuously detect the user's current blood glucose (BG) level. Generally, detection module 100 is a Continuous Glucose Monitoring (CGM) for detecting real-time BG and monitoring BG changes. The detection module 200 also includes a detection processing unit 2001. The detection processing unit 2001 is preset with an algorithm for calculating insulin amount for infusion. When the user's current blood glucose level is detected by the detection module 200, the detection processing unit 2001 calculates the insulin amount required by the user through the preset algorithm. The insulin amount required by the user is sent to infusion module 202.

The infusion module 202 includes the essential mechanical assemblies for insulin infusion and an electronic transceiver that receives the user's insulin amount information from the detection module 200. According to the current insulin infusion amount sent by the detection module 200, infusion module 202 infuses the currently required insulin into the user's body. At the same time, the infusion status of infusion module 202 can also be fed back to detection module 200 in real-time.

In the embodiment of the present invention, the algorithm for calculating the insulin infusion amount, preset in the detection processing unit 2001, is one of the classic PID algorithms, the classic MPC algorithm, the rMPC rPID algorithm or the compound artificial pancreas algorithm. The calculation method and beneficial effects of using rPID algorithm, rMPC. The algorithm or the compound artificial pancreas algorithm is described above and will not be repeated here.

The embodiment of the present invention does not limit the specific position and connection relationship of the detection module 200 and the infusion module 202, as long as the aforementioned functional conditions can be met.

As in an embodiment of the present invention, the two are electrically connected to form an integral assembly and are pasted in the same place on the user's skin. If the two modules are connected as a whole and pasted in the same position, the number of user skin pasting devices will be reduced, thereby reducing the interference of more pasted devices on user activities; at the same time, it also effectively solves the problem of poor wireless communication between separate devices, which further enhance the user experience.

As in another embodiment of the present invention, the two modules are arranged in different components and are passed on different positions of the user's skin. The detection module 100 and the infusion module 102 transmit wireless signals to realize the mutual connection.

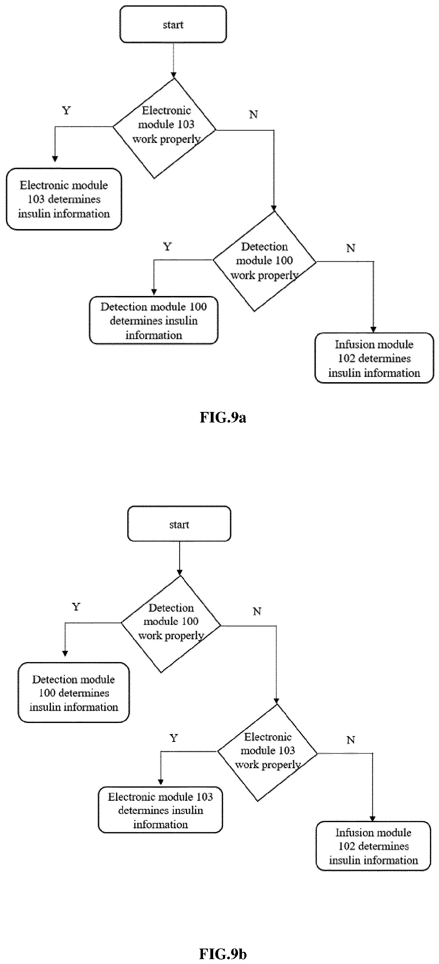

FIG.9a and FIG.9b are flow diagrams of a closed-loop artificial pancreas insulin infusion control system that determines insulin infusion information based on different priority conditions according to two other embodiments of the present invention.

In the embodiment of the invention, the closed-loop artificial pancreas insulin infusion control system mainly includes a detection module 100, an infusion module 102 and an electronic module 103.

The detection module 100 is used to continuously detect the current blood glucose value of the user. Generally, the detection module 100 is Continuous Glucose Monitoring (CGM), which can detect the user's current blood glucose value in real time and monitor the blood glucose changes, and the detection module 100 is provided with a program unit, including memory and processor, etc., and preset with a first algorithm, and the algorithm is the aforementioned classical PID algorithm, classical MPC algorithm, rMPC algorithm, rPID algorithm or compound artificial pancreas algorithm, and the detection module 100 can send the current blood glucose value to the electronic module 103 and the infusion module 102. The detection module 100 also includes a communication interface to communicate with external devices.

The infusion module 102 contains the mechanical components necessary for insulin infusion, is provided with a program unit, including memory and processor, etc., and is preset with a second algorithm, the algorithm being one of the aforementioned classical PID algorithm, classical MPC algorithm, rMPC algorithm, rPID algorithm or compound artificial pancreas algorithm, and includes a communication interface to communicate with external devices. The infusion module 102 may infuse the currently required insulin into the user according to the insulin infusion instructions. The infusion module 102 may be a conventional insulin pump or a tubeless patch insulin pump from Medtrum.

The electronic module 103 can control the operation of the detection module 100 and the infusion module 102. The electronic module 103 is a portable electronic device, such as a smart phone, PDM, smart watch, handheld, etc. The portable electronic device may include: a communication interface for communicating with the detection device, the infusion device and external remote devices, etc.; a display and display controller that may present visual information in graphics and/or text manner and control the presentation of visual information; an input device, such as a mouse, a keyboard, a touch screen, a microphone, etc., for receiving input of signals; memory and processor, etc., the memory is used for storing data, records, instructions, etc., and the processor is used to execute the instructions in the memory, control the operation of other components, etc. The processor is preset with a third algorithm, and the algorithm is one of the aforementioned classical PID algorithm, classical MPC algorithm, rMPC algorithm, rPID algorithm or compound artificial pancreas algorithm.

The infusion module 102 contains the mechanical structure necessary for insulin infusion, is provided with a program unit, including memory and processor, etc., and is pre-programmed with a second algorithm, the algorithm being one of the aforementioned classical PID algorithm, classical MPC algorithm, rMPC algorithm, rPID algorithm or composite artificial pancreas algorithm, and includes a communication interface to communicate with an external device. The root infusion module 102 may infuse the currently required insulin into the user according to the insulin infusion instructions. The infusion module 102 may be a conventional insulin pump or a catheter-less patch insulin pump from MobileU.

The electronic module 103 can control the operation of the detection module 100 and the infusion module 102. The electronic module 103 is a portable electronic device, such as a smart phone, PDM, smart watch, handheld, etc. The portable electronic device may include: a communication interface for communicating with the detection device, the infusion device and external remote devices, etc.; a display and display controller that may present visual information in graphics and/or text at the time and control the presentation of visual information; an input device, such as a mouse, a keyboard, a touch screen, a microphone, etc. keyboard, touch screen, microphone, etc., for receiving input of signals; memory and processor, etc. The memory is used to store for storing data, records, instructions, etc., and the processor is used to execute the instructions in the memory, control the operation of other components, etc. The processor is pre-programmed with a third algorithm, and the algorithm is one of the aforementioned classical PID algorithm, classical MPC algorithm, rMPC algorithm, rPID algorithm or composite artificial pancreas algorithm.

The algorithms preset in the detection module 100, the infusion module 102 and the electronic module 103 can be the same or different, and can be selected according to the needs of the actual situation.

When the closed-loop artificial pancreas insulin infusion control system starts, it will judge whether the system meets the first priority condition, and when the first priority condition is met, the first module determines the current required insulin infusion information and sends the current insulin infusion information to the infusion module 102, and the infusion module 102 carries out insulin infusion according to the infusion information; when the first priority condition is not met, it judges whether the system meets the second priority condition, when the second priority condition is met, the second module determines the current required insulin infusion information and sends the current insulin infusion information to the infusion module 102, and the infusion module 102 carries out insulin infusion in accordance with the infusion information; when both the first priority condition and the second priority condition are not met, the infusion module 102 carries out safe drug infusion in accordance with the preset insulin infusion information.

In one embodiment of the present invention, the first priority condition is that the detection module 100, the infusion module 102 and the electronic module 103 all work properly, and the first module is the electronic module 103, which determines the currently required insulin infusion information. The electronic module 103 can decide the currently required insulin infusion information alone or jointly with other modules. The electronic module 103 decides alone in the following way: after the detection module 100 sends the detected real-time blood glucose information to the electronic module 103, the electronic module 103 calculates the currently required insulin infusion information of the user through the preset algorithm and sends the insulin infusion information to the infusion module 102, and the infusion module 102 carries out drug infusion according to the insulin infusion information sent by the electronic module 103. The electronic module 103, in conjunction with other modules, determines in a manner that includes:
(1) The detection module 100 sends the real-time blood glucose information to the infusion module 102 and the electronic module 103, and the infusion module 102 and the electronic module 103 each calculate the insulin infusion information I₁ and I₂ required by the user at present according to the preset algorithms, and the infusion module 102 sends the calculated insulin infusion information I₁ to the electronic module 103, and the electronic module 103 further processes I₁ and I₂ to determine the final insulin infusion information.
(2) The detection module 100 sends the real-time blood glucose information to the electronic module 103, and the electronic module 103 calculates the insulin infusion information I₂ currently required by the user in accordance with the preset algorithm, while the detection module 100 also calculates the insulin infusion information I₁ currently required by the user at the same time in accordance with the set algorithm, and the detection module 100 sends the calculated insulin infusion information I₁ to the electronic module 103, and the electronic module 103 further processes I₁ and I₂ to determine the final insulin infusion information.
(3) The detection module 100 sends the real-time blood glucose information to the infusion module 102, and the infusion module 102 calculates the insulin infusion information I₂ currently required by the user according to the preset algorithm, and at the same time the detection module 100 also calculates the insulin infusion information I₁ currently required by the user according to the set algorithm, and the detection module 100 and the infusion module 102 send the calculated insulin infusion information I₁ and I₂ to the electronic module 103, respectively. The electronic module 103 further processes I₁ and I₂ to determine the final insulin infusion information.
   The conjunction processing mode for the electronic module 103 processing I₁ and I₂ includes previously described the optimization of the average value, the optimization of the weighted average value, and the optimization of comparison with the results of statistical analysis of historical data, which will not be repeated here.
(4) The detection module 100 sends the real-time blood glucose information to the infusion module 102 and the electronic module 103, and the infusion module 102 and the electronic module 103 each calculate the insulin infusion information I₁ and I₂ required by the user according to the preset algorithms, and the detection module 100 also calculates the insulin infusion information I₃ required by the user according to the set algorithm, and the detection module 100 and the infusion module 102 send the calculated insulin infusion information I₃ and I₁ to the electronic module 103, which further processes I₁, I₂ and I₃ to determine the final insulin infusion information. The electronic module 103 processes I₁, I₂ and I₃ in a manner similar to the previously described mean value optimization, weighted mean value optimization, and comparison optimization with the results of statistical analysis of historical data, which will not be repeated here.

When the first priority condition is not met, the system determines whether the second priority condition is met, and the second priority condition is that the electronic module 103 is not working properly while the detection module 100 is working normally, and the second module is the detection module 100 when the second priority condition is satisfied, and the detection module 100 determines the current required insulin infusion information. Here, the electronic module 103 does not work properly can be the case that the electronic module 103 is not able to send and receive information because the location of the electronic module 103 is beyond the normal use, or the software failure such as the failure of the control unit so that the calculation cannot be performed, or other physical failure of the electronic module 103, etc. The detection module 100 may determine the currently required insulin infusion information alone, or it may determine the currently required insulin infusion information jointly with other modules. The way the detection module 100 decides alone is: when the detection module 100 calculates the insulin infusion information currently required by the user through the preset algorithm based on the real-time blood glucose information detected by itself, and sends the insulin infusion information to the infusion module 102, the infusion module 102 carries out drug infusion according to the insulin infusion information sent by the detection module 100. The detection module 100 jointly decides with other modules in the following manner: the detection module 100 sends the real-time blood glucose information to the infusion module 102, the infusion module 102 calculates the insulin infusion information I₂ currently required by the user according to the preset algorithm, and at the same time the detection module 100 also calculates the insulin infusion information I₁ currently required by the user according to the set algorithm, and the infusion module 102 sends the calculated insulin information I₂ to the detection module 100, and the detection module 100 further processes I₁ and I₂ to determine the final insulin infusion information. The way the detection module 100 processes I₁ and I₂ includes previously described the optimization of the average value, the optimization of the weighted average value, and the optimization of the comparison with the results of statistical analysis of historical data, which will not be repeated here.

In another embodiment of the present development, the first priority condition is that the detection module 100, the infusion module 102 and the electronic module 103 are all work properly, the first module is the detection module 100, and the detection module 100 determines the currently required insulin infusion information. The detection module 100 can decide the currently required insulin infusion information alone or jointly with other modules. The detection module 100 decides alone in the following way: when the detection module 100 calculates the currently required insulin infusion information of the user through the preset algorithm based on the detected real-time blood glucose information and sends the insulin infusion information to the infusion module 102, the infusion module 102 carries out drug infusion according to the insulin infusion information sent by the detection module 100. The detection module 100, in conjunction with other modules, determines in a manner that includes:
(1) the detection module 100 sends the real-time blood glucose information to the infusion module 102, the infusion module 102 calculates the insulin infusion information I₂ currently required by the user according to the preset algorithm, and at the same time the detection module 100 also calculates the insulin infusion information I₁ currently required by the user according to the set algorithm at the same time, the infusion module 102 sends the calculated insulin infusion information I₂ to the detection module 100, and the detection module 100 further processes I₁ and I₂ to determine the final insulin infusion information.
(2) The detection module 100 sends the real-time blood glucose information to the electronic module 103, and the electronic module 103 calculates the insulin infusion information I₂ currently required by the user according to the preset algorithm, while the detection module 100 also calculates the insulin infusion information I₁ currently required by the user according to the set algorithm at the same time, and the electronic module 103 sends the calculated insulin infusion information I₂ to the detection module 100, and the detection module 100 further processes I₁ and I₂ to determine the final insulin infusion information.
(3) The detection module 100 sends the real-time blood glucose information to the infusion module 102 and the electronic module 103, and the infusion module 102 and the electronic module 103 calculates the insulin infusion information I₁ and I₂ required by the user at present according to the preset algorithms, and the infusion module 102 and the electronic module 103 sends the calculated insulin infusion information I₁ and I₂ to the detection module 100, respectively, and the detection module 100 then further processes I₁ and I₂ to determine the final insulin infusion information.
   The conjunction processing mode for the detection module 100 processing I₁ and I₂ includes previously described the optimization of the average value, the optimization of the weighted average value, and the optimization of comparison with the results of statistical analysis of historical data, which will not be repeated here.
(4) The detection module 100 sends the real-time blood glucose information to both the infusion module 102 and the electronic module 103, and the infusion module 102 and the electronic module 103 calculate the user's current required insulin infusion information I₁ and I₂ , respectively, according to the preset algorithms, while the detection module 100 also calculates the user's current required insulin infusion information I₃ at the same time according to the set algorithm, and the electronic module 103 and the infusion module 102 send the calculated insulin infusion information I₂ and I₁ to the detection module 100 respectively, and the detection module 100 further processes I₁, I₂ and I₃ to determine the final insulin infusion information. The detection module 100 processes I₁, I₂ and I₃ in a manner similar to the previously described mean value optimization, weighted mean value optimization, and comparison optimization with the results of statistical analysis of historical data, which will not be repeated here.

When the first priority condition is not satisfied, the system determines whether the second priority condition is satisfied, and the second priority condition is that the electronic module 103 works normally while the detection module 100 does not work normally, and the second module is the electronic module 103 when the second priority condition is satisfied, and the electronic module 103 instructs the infusion module 102 to carry out safe drug infusion according to the preset insulin infusion information. Here, the detection module 100 does not work properly at least including the detection module 103 cannot properly detect the current blood glucose value of the user in real time or cannot properly send and receive information.

It should be further noted that in other embodiments of the present invention, the closed-loop artificial pancreas insulin infusion control system may not decide insulin infusion information according to the priority conditions, but may decide the final insulin infusion information directly according to the above-mentioned electronic module 103 alone or jointly decide way, or decide the final insulin infusion information directly according to the above-mentioned detection 100 alone or jointly decide way as previously described, which will not be repeated here.

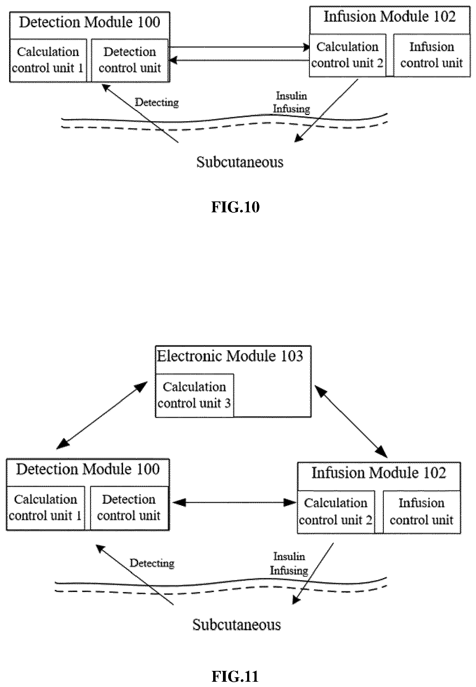

FIG.10 is a schematic diagram of the module relationship of the closed-loop artificial pancreas insulin infusion control system according to another embodiment of the present invention.

In the embodiment of the invention, the closed-loop artificial pancreas insulin infusion control system mainly includes a detection module 100 and an infusion module 102.

The detection module 100 is used to continuously detect the current blood glucose value of the user. Generally, the detection module 100 is Continuous Glucose Monitoring (CGM), which can detect the user's current blood glucose value in real time and monitor the blood glucose changes. The detection module 100 includes a communication interface to communicate with external devices. The detection module 100 is further provided with a calculation program unit 1, including memory and processor, etc., storing information such as the user's historical physical status, blood glucose value and insulin infusion dose, and preset with a first algorithm, and the algorithm is one of the aforementioned classical PID algorithm, classical MPC algorithm, rMPC algorithm, rPID algorithm or compound artificial pancreas algorithm. When the calculation control unit 1 is activated, the calculation control unit 1 may calculate insulin infusion information I₁ based on the detected current blood glucose value, and send the calculated insulin infusion amount information I₁ to an external device, and at the same time, it also can receive information from the external device and perform further processing of the information, such as receiving insulin infusion information I₂ sent by the infusion module 102 and performing further processing for I₁ and I₂. Here, the further processing means include the optimization methods described above, such as mean value optimization, weighted mean value optimization, and comparison optimization with the results of statistical analysis of historical data, such as selecting insulin infusion information that is closer to the results of statistical analysis, or the mean or weighted mean value of historical information, and the processed information can also be sent to an external device. In addition, since the history information of the user is also stored in the detection module 100, the calculation control unit 1 can also further process the insulin infusion information I₁ calculated by the detection module 100 and the history information I₄, or receive the insulin infusion information I₂ sent by the infusion module 102 and further process the insulin infusion information I₂ and the history information I₄, where the further processing includes, but is not limited to, for example, selecting the mean or weighted mean value of the insulin infusion information I₁ or I₂ and the historical information I₄ as the final insulin infusion information. A detection control unit is provided in the detection module 100 for controlling the detection module 100 for blood glucose detection and communicating with the external device to send the detected current blood glucose value to the external device.

The infusion module 102 contains the mechanical components necessary for insulin infusion, and includes a communication interface to communicate with external devices. The infusion module 100 is further provided with a calculation program unit 2, including memory and processor, etc., storing information such as the user's historical physical status, blood glucose value and insulin infusion dose, and preset with a second algorithm, and the algorithm is one of the aforementioned classical PID algorithm, classical MPC algorithm, rMPC algorithm, rPID algorithm or compound artificial pancreas algorithm. When the calculation control unit 2 is activated, the calculation control unit 2 may calculate insulin infusion information I₂ based on the current blood glucose value detected by the detection module 100, and send the calculated insulin infusion amount information I₁ to an external device, and at the same time, it also can receive information from the external device and perform further processing of the information, such as receiving insulin infusion information I₁ sent by the detection module 100 and performing further processing for I₁ and I₂. Here, the further processing methods include the optimization methods described above, such as mean value optimization, weighted mean value optimization, and comparison optimization with the results of statistical analysis of historical data, such as selecting insulin infusion information that is closer to the results of statistical analysis, or the mean or weighted mean value of historical information, and the processed information can also be sent to an external device. In addition, since the history information of the user is also stored in the infusion module 102, the calculation control unit 2 can also further process the insulin infusion information I₂ calculated by the infusion module 100 and the history information I₄, or receive the insulin infusion information I₁ sent by the detection module 100 and further process the insulin infusion information I₁ and the history information I₄, where the further processing includes, but is not limited to, for example, selecting one of the insulin infusion information I₁ or I₂ and the historical information I₄ or the mean or weighted mean value of the insulin infusion information I₁ or I₂ and the historical information I₄ as the final insulin infusion information. An infusion control unit is provided in the infusion module 102 for receiving the insulin infusion information and controlling the infusion module 102 to infuse the currently required insulin into the user according to the insulin infusion instructions. The infusion module 102 may be a conventional insulin pump or a tubeless patch insulin pump from Medtrum.

In this embodiment of the invention, at least one calculation control unit is provided in the closed-loop artificial pancreas insulin infusion control system, or at least one calculation control unit is activated. At this time, the current required insulin information of the user is determined by one of the modules in the closed-loop artificial pancreatic insulin infusion control system. When there is only one calculation control unit or only one calculation control unit is activated in the closed-loop artificial pancreas insulin infusion control system, the insulin infusion amount information is determined by the module with the calculation control unit or the module in which the calculation control unit is activated. For example, when only the calculation control unit 1 set in the detection module 100 or only the calculation control unit 1 set in the detection module 100 is activated, the calculation control unit 1 calculates insulin infusion information I₁ based on the detected current blood glucose value and sends the insulin infusion information I₁ to the infusion module 102, and the infusion control unit in the infusion module 102 receives the insulin infusion amount information I₁ and controls the infusion module 102 to infuse insulin into the user according to the insulin infusion information I₁; or the detection module 100 further processes the insulin infusion information I₁ calculated by itself and the history information I₄. When only the calculation control unit 2 set in the infusion module 102 or only the calculation control unit 2 set in the infusion module 102 is activated, the calculation control unit 2 receives the current blood glucose value detected by the detection module 100 and calculates insulin infusion information I₂ based on the current blood glucose value according to the preset second algorithm, and the infusion module 102 controls the infusion module 102 to infuse insulin into the user's body based on the insulin infusion information I₂; or the infusion module 102 further processes the insulin infusion information I₂ calculated by itself and the historical information I₄.

When there are two calculation control units set in the closed-loop artificial pancreas insulin infusion control system and both calculation control units are activated, the current insulin infusion information can be determined by either module according to the actual demand. The current insulin information is determined either independently or jointly. The independent determination method is that the module calculates the current insulin infusion information required by the user based on the current blood glucose value detected by the detection module 100 through the algorithm preset in the module, or further processes the insulin infusion information calculated by itself and the historical information. The joint determination method is that the two modules calculate the current insulin infusion information required by the user based on the current blood glucose value detected by the detection module 100 through the preset algorithms in their modules, respectively, while the module that does not determine the current insulin infusion information sends the calculated current insulin information to the module that determines the current insulin infusion information, and the module that determines the current insulin infusion information further processes the two current insulin information to determine the current insulin infusion information required by the end user or the module that determines the current insulin infusion information receives the insulin infusion information calculated by the other module and compares it with the historical insulin infusion information. The module that determines the current insulin infusion information further processes the two current insulin information to determine the current insulin infusion information required by the end user, or the module that determines the current insulin infusion information receives the insulin infusion information calculated by the other module and further processes it with the historical information. Such as, when it is determined by the detection module 100, the detection module 100 may determine the currently required insulin infusion information independently, or it may determine the currently required insulin infusion information jointly with the infusion module 102. The independent determination method of the detection module 100 is that: when the detection module 100 calculates the insulin infusion information currently required by the user through the preset algorithm based on the detected real-time blood glucose information and sends the insulin infusion information to the infusion module 102, the infusion module 102 performs drug infusion according to the insulin infusion information sent by the detection module 100; or the detection module 100 calculates the insulin infusion information currently required by the user through the preset algorithm based on the detected real-time blood glucose information and further processes it and the historical information. The joint determination method of the detection module with the other modules is that the detection module 100 sends the real-time blood glucose information to the infusion module 102, and the infusion module 102 calculates the insulin infusion information I₂ currently required by the user according to the preset algorithm, while the detection module 100 also calculates the insulin infusion information I₁ currently required by the user according to the set algorithm, and the infusion module 102 then sends the calculated insulin infusion information I₂ to the detection module 100, and the detection module 100 further processes I₁ and I₂ to determine the final insulin infusion information; or the detection module 100 receives the insulin infusion information I₂ calculated by the infusion module 102 and further processes it with the historical information. That is, this joint determination method is: the detection module 100 and the infusion module 102 perform calculation at the same time, but the detection module 100 performs further processing to finally decide the insulin infusion information; or only the infusion module 102 perform calculation, and the detection module 100 further processes it with the historical information to finally decide the insulin infusion information.

Such as, when it is determined by the infusion module 102, the infusion module 102 may determine the currently required insulin infusion information independently, or it may determine the currently required insulin infusion information jointly with the detection module 100. The independent determination method of the infusion module 102 is that: when the infusion module 102 receives the current blood glucose value detected by the detection module 100, and calculates the insulin infusion information currently required by the user through the preset algorithm based on the detected real-time blood glucose information, the infusion module 102 performs drug infusion according to the calculated insulin infusion information; or infusion module 102 calculates the insulin infusion information currently required by the user through the preset algorithm based on the real-time blood glucose information detected by the detection module and further processes it with the historical information. The joint determination method of the infusion module with the other modules is that the detection module 100 sends the real-time blood glucose information to the infusion module 102, and the infusion module 102 calculates the insulin infusion information I₂ currently required by the user in accordance with the preset algorithm, while the detection module 100 also calculates the insulin infusion information I₁ currently required by the user in accordance with the set algorithm, and the detection module 100 sends the calculated insulin infusion information I₁ to the infusion module 102, and the infusion module 102 then further processes I₁ and I₂ to determine the final insulin infusion information; or the infusion module 102 receives the insulin infusion information I₁ calculated by the detection module 100 and further processes it with the historical information. That is, this joint determination method is: the detection module 100 and the infusion module 102 perform calculation at the same time, but the infusion module 102 performs further processing to finally decide the insulin infusion information; or only the detection module 100 performs calculation, and the infusion module 102 performs further processing with the historical information to finally decide the insulin infusion information.

In the embodiment of the invention, the closed-loop artificial pancreatic insulin infusion control system further comprises an electronic module 103, which can communicate with the detection module 100 and the infusion module 102 and control their operation. The electronic module 103 is a portable electronic device, such as a smart phone, PDM, smart watch, handheld, etc., and the portable electronic device is provided with a calculation control unit 3, including memory and processor, etc., and is preset with a third algorithm, and the algorithm is one of the aforementioned classical PID algorithm, classical MPC algorithm, rMPC algorithm, rPID algorithm or compound artificial pancreas algorithm, and when the calculation control unit 3 is activated, the calculation control unit 3 may calculate insulin infusion information I₃ based on the detected current blood glucose value according to the preset third algorithm and send the calculated insulin infusion information I₃ to the external device, at the same time, it also receive information from the external device and further process the information, such as receiving insulin infusion information I₂ sent by the infusion module 102 and/or the insulin infusion information I₁ calculated by detection module 100, and further processing of I₁ and/or I₂ with I₃. Here, the further processing means include the optimization methods described above, such as mean value optimization, weighted mean value optimization, and comparison optimization with the results of statistical analysis of historical data, such as selecting insulin infusion information that is closer to the results of statistical analysis, or the mean or weighted mean value of historical information, and the processed information can also be sent to an external device. In addition, since the history information of the user is also stored in the electronic module 103, the calculation control unit 3 can also further process the insulin infusion information I₃ calculated by the electronic module 103 with the history information I₄, or accept the insulin infusion information I₂ sent by the infusion module 102 and/or the insulin infusion information I₁ calculated by the detection module 100, and further processing the insulin infusion information I₂ or insulin infusion information I₁ with the history information I₄, where the further processing includes, but is not limited to, for example, selecting one of the insulin infusion information I₁ or I₂ and the history information I₄ as the final insulin infusion information, or selecting the mean or weighted mean value of the insulin infusion information I₁ or I₂ and the historical information I₄ as the final insulin infusion information. The memory is used for storing data, records, instructions, information such as the user's historical physical status, blood glucose value and insulin infusion amount, etc. The processor is used to execute the instructions in the memory and control the operation of other components, etc. The electronic module 103 may also include: a communication interface for communicating with the detection device, the infusion device and the external remote devices, etc.; a display and display controller that may present visual information in graphics and/or text and control the presentation of the visual information; an input device, such as a mouse, keyboard, touch screen, microphone, etc., for receiving the input of signals.

The algorithms preset in the detection module 100, the infusion module 102 and the electronic module 103 can be the same or different, and can be selected according to the needs of the actual situation.

In this embodiment of the invention, when the calculation control unit 3 in the electronic module 103 is activated, the current insulin infusion information required by the user may be determined by any of the activated modules of the system. The decision may be made independently or jointly.

When the decision is made by the electronic module 103, the independent determination method is as follows: when the detection module 100 sends the detected real-time blood glucose information to the electronic module 103, the electronic module 103 calculates the current insulin infusion information required by the user through the preset algorithm and sends the insulin infusion information to the infusion module 102, and the infusion module 102 performs the drug infusion in accordance with the insulin infusion information sent by the electronic module 103; or the electronic module 103 calculates the insulin infusion information currently required by the user according to the real-time blood glucose information detected by the detection module 100 through the preset third algorithm and further processes it with the historical information. At this time, although at least one calculation control unit in the detection module 100 and the infusion module 102 is activated, it does not perform calculation or further processing for the information.

The joint determination methods of the electronic module 103 with other modules include following:
(1) when the calculation control unit 2 of the infusion module 102 in the system is activated: the detection module 100 sends the real-time blood glucose information to the infusion module 102 and the electronic module 103, and the infusion module 102 and the electronic module 103 each calculate the insulin infusion information I₂ and I₃ currently required by the user according to the preset algorithm, respectively, and the infusion module 102 sends the calculated insulin infusion information I₂ to the electronic module 103, and the electronic module 103 further processes I₂ and I₃ to determine the final insulin infusion information. That is, this joint determination method is that the electronic module 103 and the infusion module 102 perform calculation at the same time, but the electronic module 103 performs further processing to finally decide the insulin infusion information.
(2) When the calculation control unit 1 set in the detection module 100 in the system is activated: the detection module 100 sends the real-time blood glucose information to the electronic module 103, and the electronic module 103 calculates the insulin infusion information I₃ currently required by the user according to the preset algorithm, while the detection module 100 also calculates the insulin infusion information I₁ currently required by the user at the same time according to the set algorithm, and the detection module 100 sends the calculated insulin infusion information I₁ to the electronic module 103, and the electronic module 103 further processes I₁ and I₃ to determine the final insulin infusion information. That is, the joint determination method is that the electronic module 103 and the detection module 100 perform calculation at the same time, but the electronic module 103 performs further processing to finally decide the insulin infusion information.
(3) When the calculation control unit 1 of the detection module 100 and the calculation control unit 2 of the infusion module 102 are activated: the detection module 100 sends the real-time blood glucose information to the infusion module 102, the infusion module 102 calculates the insulin infusion information I₂ currently required by the user according to the preset algorithm, and at the same time the detection module 100 also calculates the insulin infusion information I₁ currently required by the user according to the set algorithm. The detection module 100 and the infusion module 102 send the calculated insulin infusion information I₁ and I₂ to the electronic module 103 respectively, and the electronic module 103 further processes I₁ and I₂ to determine the final insulin infusion information. That is, this joint determination method is that the detection module 100 and the infusion module 102 participate calculation at the same time, but only the electronic module 103 performs further processing to finally decide the insulin infusion information.
(4) When the calculation control unit 1 of detection module 100 and calculation control unit 2 of infusion module 102 active at the same time in the system: detection module 100 sends real-time blood glucose information to the infusion module 102 and the electronic module 103, and the infusion module 102 and the electronic module 103 each calculate the user's current required insulin infusion information I₂ and I₃, at the same time, the detection module 100 also calculates the current insulin infusion information I₁ required by the user according to the preset algorithm. The detection module 100 and the infusion module 102 send the calculated insulin infusion information I₁ and I₂ to the electronic module 103 respectively, and the electronic module 103 further processes I₁, I₂ and I₃ to determine the final insulin infusion information. That is, the joint determination method is that the detection module 100, the electronic module 103 and the infusion module 102 perform calculation at the same time, but only the electronic module 103 performs further processing to finally decide the insulin infusion information.
(5) When at least one of the calculation control unit 1 of the detection module 100 and the calculation control unit 2 of the infusion module 102 is activated in the system, the detection module 100 or the infusion module 102 calculates the insulin infusion information I₁ or I₂ currently required by the user according to the preset algorithm, respectively, and the electronic module 103 receives the insulin infusion information I₁ or I₂ and processes it with the historical information for further processing.

When the decision is made by the detection module 100, the determination method also includes an independent determination method or a joint determination method, and the independent determination method includes the aforementioned method without the electronic module, and also includes that after the detection module 100 calculates the insulin infusion information I₁ currently required by the user respectively according to a preset algorithm and then further processes it with the historical information, i.e. at this time, although the calculation control unit 3 of the electronic module 103 is activated, it does not perform calculation of insulin infusion information, and also does not further process the calculation results.

The joint determination methods of the detection module 100 with other modules include following:
(1) When only the calculation control units of the detection module 100 and the electronic module 103 are activated in the system: the detection module 100 sends the real-time blood glucose information to the electronic module 103, and the electronic module 103 calculates the insulin infusion information I₃ currently required by the user according to the preset algorithm, while the detection module 100 also calculates the insulin infusion information currently required by the user according to the preset algorithm at the same time. The electronic module 103 sends the calculated insulin infusion information I₃ to the detection module 100, and the detection module 100 further processes I₁ and I₃ to determine the final insulin infusion information. That is, the joint determination method is that the electronic module 103 and the detection module 100 perform calculation at the same time, but only the detection module 100 performs further processing to finally decide the insulin infusion information.
(2) When the calculation control unit 2 of the infusion module 102 is also activated in the system: the detection module 100 sends the real-time blood glucose information to the infusion module 102, and the infusion module 102 calculates the insulin infusion information I₂ currently required by the user according to the preset algorithm, while the detection module 100 also calculates the insulin infusion information I₁ currently required by the user at the same time according to the preset algorithm, and the infusion module 102 sends the calculated insulin infusion information I₂ to the detection module 100, and the detection module 100 further processes I₁ and I₂ to determine the final insulin infusion information. That is, the joint determination method is that the infusion module 102 and the detection module 100 perform calculation at the same time, but only the detection module 100 performs further processing to finally decide the insulin infusion information.
(3) When the calculation control unit 2 of the infusion module 102 is also activated in the system: the detection module 100 sends the real-time blood glucose information to both the infusion module 102 and the electronic module 103, and the infusion module 102 and the electronic module 103 each calculate the insulin infusion information I₂ and I₃ required by the user at present according to the preset algorithm, respectively, and the electronic module 103 and the infusion module 102 send the calculated insulin infusion information I₃ and I₂ to the detection module 100, respectively, and then the detection module 100 further processes I₂ and I₃ to determine the final insulin infusion information. That is, this joint determination method is that the electronic module 103 and the infusion module 102 perform calculation at the same time, but only the detection module 100 performs further processing to finally decide the insulin infusion information.
(4) When the calculation control unit 2 of the infusion module 102 is also active in the system: the detection module 100 sends the real-time blood glucose information to both the infusion module 102 and the electronic module 103, and the infusion module 102 and the electronic module 103 each calculate the insulin infusion information I2 and I3 required by the user respectively according to the preset algorithm, while the detection module 100 also calculates the insulin infusion information required by the user according to the set algorithm at the same time. The electronic module 103 and the infusion module 102 send the calculated insulin infusion information I3 and I2 to the detection module 100 respectively, and the detection module 100 further processes I1, I2 and I3 to determine the final insulin infusion information. That is, the joint determination method is that the infusion module 102, the electronic module 103 and the detection module 100 perform at the same time, but the detection module 100 performs further processing to finally decide the insulin infusion information.
(5) When at least one of the calculation control unit 3 of the electronic module 103 and the calculation control unit 2 of the infusion module 102 is activated in the system, the electronic module 103 or the infusion module 102 calculates the insulin infusion information I₃ or I₂ currently required by the user respectively according to the preset algorithm, and the detection module 100 receives the insulin infusion information I3 or I₂ and processes it with the historical information for further processing.

When the decision is made by the infusion module 102, the determination method also includes an independent determination method or a joint determination method, and the determination method includes the aforementioned method without the electronic module, and also includes the infusion module 102 calculating the insulin infusion information I2 currently required by the user respectively according to the preset algorithm and then further processing it with the historical information, i.e. at this time, the calculation control unit 3 of the electronic module 103, although is activated, but it does not perform calculation of insulin infusion information, and it also does not further process the calculation results.

The joint determination methods of the infusion module 102 with other modules include following:
(1) When only the calculation control units of the infusion module 102 and the electronic module 103 are activated in the system: the detection module 100 sends real-time blood glucose information to both the infusion module 102 and the electronic module 103, and the infusion module 102 and the electronic module 103 each calculate the insulin infusion information I₂ and I₃ required by the user according to the preset algorithm, respectively, and the electronic module 103 sends the calculated insulin infusion information I₃ to the infusion module 102, and the infusion module 102 further processes I₂ and I₃ to determine the final insulin infusion information. That is, this joint determination method way is that the electronic module 103 and the infusion module 102 participate in the calculation at the same time, but only the infusion module 102 performs further processing to finally decide the insulin infusion information.
(2) When the calculation control unit 2 of the detection module 100 is also activated in the system: the detection module 100 sends the real-time blood glucose information to the infusion module 102, and the infusion module 102 calculates the insulin infusion information I₂ currently required by the user according to the preset algorithm, while the detection module 100 also calculates the insulin infusion information I₁ currently required by the user at the same time according to the preset algorithm, and the detection module 100 sends the calculated insulin infusion information I₁ to the infusion module 102, and the infusion module 102 further processes I₁ and I₂ to determine the final insulin infusion information. That is, joint determination method is that the detection module 100 and the infusion module 102 perform calculation at the same time, but only the infusion module 102 performs further processing to finally decide the insulin infusion information.
(3) When the calculation control unit 2 of the infusion module 102 in the system is also activated: the detection module 100 sends the real-time blood glucose information to the electronic module 103, and the detection module 100 and the electronic module 103 each calculate the insulin infusion information I₁ and I₃ required by the user at present according to the preset algorithm, and the detection module 100 and the electronic module 103 send the calculated insulin infusion information I₁ and I₃ to the detection infusion module 102, and the infusion module 102 then further processes I₁ and I₃ to determine the final insulin infusion information. That is, the joint determination method is that the electronic module 103 and the infusion module 100 perform calculation at the same time, but the infusion module 102 performs further processing to finally decide the insulin infusion information.
(4) When the calculation control unit 2 of the infusion module 102 is also activated in the system: the detection module 100 sends the real-time blood glucose information to both the infusion module 102 and the electronic module 103, and the infusion module 102 and the electronic module 103 each calculate the insulin infusion information I₂ and I₃ currently required by the user, respectively, according to the preset algorithm, while the detection module 100 also calculates the insulin infusion information I₁ currently required by the user simultaneously according to the preset algorithm. The electronic module 103 and the detection module 100 send the calculated insulin infusion information I₃ and I₁, respectively, to the infusion module 102, and the infusion module 102 further processes I₁, I₂ and I₃ to determine the final insulin infusion information. That is, this joint determination method is that the infusion module 102, the electronic module 103 and the detection module 100 perform calculation at the same time, but only the infusion module 102 performs further processing to finally decide the insulin infusion information.
(5) When at least one of the calculation control unit 1 of the detection module 100 and the calculation control unit 3 of the electronic module 103 is also activated in the system, the detection module 100 or the electronic module 103 calculates the insulin infusion information I₁ or I₃ currently required by the user, respectively, according to the preset algorithm, and the infusion module 102 receives the insulin infusion information I₁ or I₃ and processes it with the historical information for further processing.

In the embodiment of the present invention, the further processing with the historical information when only one calculation control unit performs the calculation includes, but is not limited to, selecting one of the insulin infusion information calculated by the calculation control unit and the historical information as the final insulin infusion information, or selecting the average or weighted average value of the insulin infusion information by the calculation control unit and the historical information calculated as the final insulin infusion information. When there are two calculation units perform calculation, the further processing means include the optimization methods described above, such as mean value optimization, weighted mean value optimization, and comparison optimization with the results of statistical analysis of historical data, such as selecting insulin infusion information that is closer to the results of statistical analysis, or the mean or weighted mean value of historical information.

FIG.11 is a schematic diagram of the module relationship of the closed-loop artificial pancreas insulin infusion control system according to another embodiment of the present invention.

In another embodiment of the present invention, the closed-loop artificial pancreas insulin infusion control system includes a detection module 100, an infusion module 102 and an electronic module 103. A detection control unit is provided within the detection module 100 for controlling the detection module 100 for blood glucose detection and communicating with an external device. An infusion control unit is provided within the infusion module 102 for receiving insulin infusion information and controlling the infusion module 102 to infuse the currently required insulin into the user's body according to the insulin infusion information; in addition, all the three modules can be provided with a calculation control unit and a preset algorithm, and calculate the insulin information based on the current blood glucose value detected by the detection module and further process the multiple insulin infusion information at the same time, and at least one of the three modules is provided with a calculation control unit or at least one calculation control unit is activated. Other components or functions of the three modules are as previously described and will not be repeated here.

When only one calculation control unit is set among the three modules or when more than one calculation control units are set but only one calculation control unit is activated, the module in which the calculation control unit is set or the calculation control unit is activated determines the current insulin infusion information required by the user individually. The independent determination method of the detection module 100, the infusion module 102 or the electronic module 103 is that the detection module 100, the infusion module 102 or the electronic module 103 calculate the insulin infusion information required by the user based on the blood glucose information detected by the detection module 100 through their respective preset algorithms, respectively or the detection module 100, the infusion module 102 or the electronic module 103 further process the respective calculated insulin infusion information required by the user based on the blood glucose the detection module 100 n with the historical information.

When only two calculation control units are provided among the three modules or when more than one is provided but only two calculation control units are activated, then the current insulin infusion information required by the user may be determined by any one of the modules in which the calculation control unit is activated, and the determination method includes an independent determination method or a joint determination method. The independent determination method is as described previously, at that time, though there is another calculation control unit active in the system, but it does not perform calculation or further processing of the information. The joint determination method includes is that both of the two calculation control units are perform calculation or further processing of the information, specifically in the following ways:
(1) the calculation control units in the detection module 100 and the infusion module 102 are activated, and both perform calculation, and the current insulin infusion information required by the user is finally determined by the detection module 100 after further processing;
(2) the calculation control units in the detection module 100 and the infusion module 102 are activated, and both perform calculation, and the current insulin infusion information required by the user is finally determined by the infusion module 102 after further processing
(3) the calculation control units in the detection module 100 and the electronic module 103 are activated, and both perform calculation, and the final decision on the current insulin infusion information required by the user is made by the detection module 100 after further processing.
(4) the calculation control units in the detection module 100 and the electronic module 103 are activated, and both perform calculation, and the final decision on the current insulin infusion information required by the user is made by the detection module 100 after further processing.
(5) the calculation control units in the infusion module 102 and the electronic module 103 are activated, and both perform calculation, and the final decision on the current insulin infusion information required by the user is made by the electronic module 103 after further processing.
(6) the calculation control units in the infusion module 102 and the electronic module 103 are activated, and both perform calculation, and the final decision on the current insulin infusion information required by the user is made by the infusion module 102 after further processing.

When the determination is jointly made, it further includes a way in which one calculation control unit is performs calculation and the other calculation control unit performs further processing, specifically including the following ways:
(1) the calculation control units in the detection module 100 and the infusion module 102 are activated, and only the infusion module 102 performs calculation, and the final decision on the current insulin infusion information required by the user is made by the detection module 100 after further processing.
(2) the calculation control units in the detection module 100 and the infusion module 102 are activated, and only the infusion module 102 performs calculation, and the final decision on the current insulin infusion information required by the user is made by the infusion module 102 after further processing.
(3) the calculation control units in the detection module 100 and the electronic module 103 are activated, and only the electronic module 103 performs calculation, and the final decision on the current insulin infusion information required by the user is made by the detection module 100 after further processing.
(4) the calculation control units in the detection module 100 and the electronic module 103 are activated, and only the electronic module 103 performs calculation, and the final decision on the current insulin infusion information required by the user is made by the electronic module 103 after further processing.
(5) the calculation control units in the infusion module 102 and the electronic module 103 are activated, and only the infusion module 102 performs calculation, and the final decision on the current insulin infusion information required by the user is made by the electronic module 103 after further processing.
(6) the calculation control units in the infusion module 102 and the electronic module 103 are activated, and only the infusion module 102 performs calculation, and the final decision on the current insulin infusion information required by the user is made by the infusion module 102 after further processing.

When all three calculation control units in the system are activated and all performs or further processing, the joint determination method includes:
(1) the detection module 100, the infusion module 102, and the electronic module 103 are all perform calculation, and the final decision on the current insulin infusion information required by the user is made by the detection module 100 after further processing.
(2) the detection module 100, the infusion module 102, and the electronic module 103 are all perform calculation, and the final decision on the current insulin infusion information required by the user is made by the infusion module 102 after further processing.
(3) the detection module 100, the infusion module 102, and the electronic module 103 are all perform calculation, and the final decision on the current insulin infusion information required by the user is made by the electronic module 103 after further processing.
(4) the infusion module 102 and the electronic module 103 are all perform calculation, and the final decision on the current insulin infusion information required by the user is made by the detection module 100 after further processing.
(5) the detection module 100 and the electronic module 103 are all perform calculation, and the final decision on the current insulin infusion information required by the user is made by the infusion module 102 after further processing.
(6) the detection module 100 and the infusion module 102 are all perform calculation, and the final decision on the current insulin infusion information required by the user is made by the electronic module 103 after further processing.

When the above two or three calculation control units are activated, the joint determination method of the current insulin infusion information required by the user is as described in the previous embodiment and will not be repeated here.

As previously mentioned, in embodiments of the present invention, when only one calculation control unit performs calculation, the further processing means with historical information includes, but is not limited to, selecting one of the insulin infusion information calculated by the calculation unit and the historical information or the mean or weighted mean value of the insulin infusion information calculated by the calculation unit and the historical information as the final insulin infusion information. When at least two calculation control units perform calculation, the further processing means with historical information includes, but is not limited to, the further processing means include the optimization methods described above, such as mean value optimization, weighted mean value optimization, and comparison optimization with the results of statistical analysis of historical data, such as selecting insulin infusion information that is closer to the results of statistical analysis, or the mean or weighted mean value of historical information.

In this embodiment of the invention, calculations and/or further processing are performed when one of the calculation control units in the system is activated. Preferably, when the electronic module 103, such as the handheld, is within the normal use distance, only the calculation control unit 3 within the electronic module 103 is activated to determine the current insulin infusion information alone, which can avoid the detection module 100 and the infusion module 102 performing calculation, thereby reducing their power consumption, extending their service life, reducing the frequency of user replacement and enhancing the user experience. Or at least one calculation control unit in the detection module 100 or infusion module 102 is also activated, and multiple modules perform calculation, and the final decision on insulin infusion information is made by the electronic module 103, which improves the calculation accuracy of insulin infusion information while reducing the power consumption of the detection module 100 or infusion module 102. Or, according to the actual situation, switch the calculation control unit in the electronic module 103, detection module 100 or infusion module 102 to increase the flexibility of system, so that the closed-loop artificial pancreatic insulin infusion control system always operates normally while maintaining the best state.

When the electronic module 103, such as a handheld, is not within the normal use range, or when the calculation control unit 3 within the electronic module 103 is disabled, activating at least one calculation control unit of the detection module 100 or the infusion module 102, and selecting one to determine the insulin infusion amount according to the actual situation, such as switching the activation of the calculation control unit according to the power remaining in the detection module 100 and the infusion module 102, independently or jointly determined the insulin infusion information, without being limited by the electronic module 103, increasing the flexibility of system and making the closed-loop artificial pancreatic insulin infusion control system always function properly while maintaining the optimal state.

In summary, the present invention discloses a closed-loop artificial pancreas insulin infusion control system, the system includes a detection module and an infusion module, a detection control unit is set in the detection module, an infusion control unit is set in the infusion module, and at least one calculation control unit is set in the detection module and the infusion module for calculating insulin infusion information and/or further processing of different insulin infusion information. Therefore, the closed-loop artificial pancreas insulin infusion control system can automatically switch the control unit according to different situations to avoid affecting the user experience due to the failure of a module or even bringing safety risks to the user, and to avoid replacing the whole device when only part of the function of some components fails, and to improve the utilization rate of each component of the closed-loop artificial pancreas.

While the invention has been described in detail with reference to the specific embodiments of the present invention, it should be understood that it will be appreciated by those skilled in the art that the above embodiments may be modified without departing from the scope and spirit of the invention. The scope of the invention is defined by the appended claims.

## Claims

1. A closed-loop artificial pancreas insulin infusion control system, comprising,
a detection module, provided with a detection control unit, used to control the detection module to detect blood glucose and communicate with external devices; and
an infusion module, provided with an infusion control unit, used to control the infusion module to infuse currently needed insulin into a user's body and communicate with external devices; wherein,
the infusion module is provided with a first calculation control unit and/or the detection module is provided with a second calculation control unit, the first calculation control unit and/or the second calculation control unit are preset with algorithms for calculating insulin infusion information based on the blood glucose value detected by the detection module through the preset algorithms and/or further processing different insulin infusion information.

2. The closed-loop artificial pancreas insulin infusion control system of claim 1, wherein,
at least one of the calculation control units is activated.

3. The closed-loop artificial pancreas insulin infusion control system of claim 1, wherein,
the insulin infusion information is determined by the detection module or the infusion module.

4. The closed-loop artificial pancreas insulin infusion control system of claim 3, wherein,
the determination method of the insulin infusion information is an independent determination method or a jointly determination method.

5. The closed-loop artificial pancreas insulin infusion control system of claim 4, wherein,
the independent determination method is that the detection module or the infusion module calculates the current insulin infusion information based on the blood glucose value provided by the detection module through the preset algorithms, respectively.

6. The closed-loop artificial pancreas insulin infusion control system of claim 4, wherein,
the independent determination method is that after the detection module or the infusion module calculates the current insulin infusion information based on the blood glucose value provided by the detection module through the preset algorithms, respectively, further processing the insulin infusion information and history information.

7. The closed-loop artificial pancreas insulin infusion control system of claim 4, wherein,
the jointly determination method is that the detection module and the infusion module perform calculation of the insulin infusion information, and the detection module or the infusion module perform the further processing to determine the insulin infusion information.

8. the closed-loop artificial pancreas insulin infusion control system of claim 4, wherein,
the jointly determination method is that one of the detection module and the infusion module calculates the insulin infusion information, and the other one further processes the insulin information and historical information after receiving the insulin information.

9. The closed-loop artificial pancreas insulin infusion control system of claim 1, wherein, further includes an electronic module, provided with a third calculation control unit, which is preset with an algorithm for calculating the insulin infusion information based on the blood glucose value detected by the detection module through the preset algorithm and/or further processing different insulin infusion information.

10. The closed-loop artificial pancreas insulin infusion control system of claim 9, wherein,
one of the detection module, the infusion module or the electronic module determines the insulin infusion information.

11. The closed-loop artificial pancreas insulin infusion control system of claim 10, wherein,
the determination method of the insulin infusion information is an independent determination method or a jointly determination method.

12. The closed-loop artificial pancreas insulin infusion control system of claim 11, wherein,
the independent determination method is that the detection module, the infusion module or the electronic module calculates the current insulin infusion information based on the blood glucose value provided by the detection module through the preset algorithms, respectively.

13. The closed-loop artificial pancreas insulin infusion control system of claim 11, wherein,
the independent determination method is that after the detection module, the infusion module or the electronic module calculates the current insulin infusion information based on the blood glucose value provided by the detection module through the preset algorithms, respectively, further processing the insulin infusion information and history information.

14. The closed-loop artificial pancreas insulin infusion control system of claim 11, wherein,
the jointly determination method is that at least two modules of the electronic module, the detection module and the infusion module simultaneously calculate the insulin infusion information based on the blood glucose value provided by the detection module through their respective preset algorithms, respectively, and the module that ultimately determines the insulin infusion information performs further processing.

15. The closed-loop artificial pancreas insulin infusion control system of claim 14, wherein,
the module that ultimately determines the insulin infusion information itself also perform calculation of the insulin infusion information through the preset algorithm based on the blood glucose value provided by the detection module.

16. The closed-loop artificial pancreas insulin infusion control system of claim 14, wherein,
the module that ultimately determines the insulin infusion information itself does not perform calculation of the insulin infusion information.

17. The closed-loop artificial pancreas insulin infusion control system of claim 11, wherein,
the jointly determination method is that one of the detection module, the infusion module and the electronic module perform calculation of the insulin infusion information based on the blood glucose value detected by the detection module through the preset algorithm, and another module further processes the insulin information and historical information.

18. A closed-loop artificial pancreas insulin infusion control system, comprising,
a detection module, provided with a detection control unit, used to control the detection module to detect blood glucose and communicate with external devices; and
an infusion module, provided with an infusion control unit, used to control the infusion module to infuse currently needed insulin into a user's body and communicate with external devices; and an electronic module, communicating with the detection module and the infusion module; wherein,
at least one of the infusion module, the detection module and the electronic module is provided with a calculation control unit, which is preset with an algorithm for calculating insulin infusion information based on the blood glucose value detected by the detection module through the preset algorithm and/or further processing different insulin infusion information.

19. The closed-loop artificial pancreas insulin infusion control system of claim 18, wherein,
at least one of the calculation control units is activated.

20. The closed-loop artificial pancreas insulin infusion control system of claim 18, wherein,
one of the infusion module, the detection module and the electronic module determines the insulin infusion information, the determination method of the insulin infusion information is an independent determination method or a jointly determination method.

21. The closed-loop artificial pancreas insulin infusion control system of claim 20, wherein,
the independent determination method is that the detection module, the infusion module or the electronic module calculates the current insulin infusion information based on the blood glucose value provided by the detection module through the preset algorithms, respectively.

22. The closed-loop artificial pancreas insulin infusion control system of claim 21, wherein,
the independent determination method is that after the detection module, the detection module or the electronic module calculates the current insulin infusion information based on the blood glucose value provided by the detection module through the preset algorithms, respectively, further processing the insulin infusion information and history information.

23. The closed-loop artificial pancreas insulin infusion control system of claim 21, wherein,
the jointly determination method is that at least two modules of the electronic module, the detection module and the infusion module simultaneously calculate the insulin infusion information based on the blood glucose value provided by the detection module through their respective preset algorithms, respectively, and the module that ultimately determines the insulin infusion information performs further processing.

24. The closed-loop artificial pancreas insulin infusion control system of claim 21, wherein,
the jointly determination method is that one of the detection module, the infusion module and the electronic module perform calculation of the insulin infusion information based on the blood glucose value detected by the detection module through the preset algorithm, and another module further processes the insulin information and historical information.

25. A closed-loop artificial pancreas insulin infusion control system, comprising,
a detection module, provided with a detection control unit, used to control the detection module to detect blood glucose and communicate with external devices; and
an infusion module, provided with an infusion control unit, used to control the infusion module to infuse currently needed insulin into a user's body and communicate with external devices; and an electronic module, communicating with the detection module and the infusion module; wherein,
at least two of the infusion module, the detection module and the electronic module are provided with a calculation control unit, which is preset with an algorithm for calculating insulin infusion information based on the blood glucose value detected by the detection module through the preset algorithm and/or further processing different insulin infusion information;
the module that determines the insulin infusion information is one of the modules provided with a calculation control unit, and can be switched according to different conditions.

26. The closed-loop artificial pancreas insulin infusion control system of claim 25, wherein,
at least two of the calculation control units are activated, and the module that determines the insulin infusion information is one of the modules that the calculation control unit is activated.

27. The closed-loop artificial pancreas insulin infusion control system of any one claim 1-26, wherein,
the preset algorithm is one of a classic PID algorithm, a classic MPC algorithm, a rMPC algorithm, a rPID algorithm or a compound artificial pancreas algorithm, and the rMPC algorithm or rPID algorithm is an algorithm that converts blood glucose that is asymmetric in the original physical space to a blood glucose risk that is approximately symmetric in the risk space based on the classic PID algorithm and the classic MPC algorithm.

28. The closed-loop artificial pancreas insulin infusion control system of claim 27, wherein,
the conversion method of blood glucose risk space of the rMPC algorithm and the rPID algorithm includes one or more of a segmented weighting conversion, a relative value conversion, a blood glucose risk index conversion, and an improved control variability grid analysis conversion.

29. The closed-loop artificial pancreas insulin infusion control system of claim 28, wherein,
the blood glucose risk conversion method of the rMPC algorithm and the rPID algorithm further include one or more of the following processing methods:
① subtract a component which is proportional to the predicted plasma insulin concentration;
② deduct the amount of insulin that has not yet worked in the body;
③ the autoregressive method is used to compensate for the detecting delay of interstitial fluid glucose concentration and blood glucose concentration.

30. The closed-loop artificial pancreas insulin infusion control system of claim 29, wherein,
the compound artificial pancreas algorithm, including a first algorithm and a second algorithm, the first algorithm is used to calculate the first insulin infusion amount I₁, the second algorithm is used to calculate the second insulin infusion volume I₂, and the compound artificial pancreas algorithm further optimizes I₁ and I₂ to obtain the final insulin infusion amount I₃.

31. The closed-loop artificial pancreas insulin infusion control system of claim 30, wherein,
the final insulin infusion amount I₃ is optimized by the average value *I̅* of the first insulin infusion amount I₁ and the second insulin infusion amount I₂:
① obtain the average value *I̅* of the first insulin infusion amount I₁ and the second insulin infusion amount I₂, and $\overline{I} = \frac{{I}_{1} + {I}_{2}}{2}$;
② substitute the average value *I̅* into the first algorithm and the second algorithm to adjust the algorithm parameters;
③ recalculate the first insulin infusion amount I₁ and the second insulin infusion amount I₂ based on the current blood glucose level and the first algorithm and the second algorithm with adjusted the parameters;
④ calculate steps ①~③ cyclically until I₁=I₂, and the final insulin infusion amount I₃=I₁=I₂.

32. The closed-loop artificial pancreas insulin infusion control system of claim 25, wherein,
the final insulin infusion amount I₃ is optimized by the weighted value *I̅'̅* of the first insulin infusion amount I₁ and the second insulin infusion amount I₂:
① obtain the weighted value *I̅'̅* of the first insulin infusion amount I₁ and the second insulin infusion amount I₂, and *I̅'̅* = *α* * *I₁* + *β* _{*} *I*₂, where α and β are the weighting coefficients of the first insulin infusion amount I₁ and the second insulin infusion amount I₂, respectively.
(2) substitute the average value *I̅'̅* into the first algorithm and the second algorithm to adjust the algorithm parameters;
③ recalculate the first insulin infusion amount I₁ and the second insulin infusion amount I₂ based on the current blood glucose level and the first algorithm and the second algorithm with adjusted the parameters;
④ calculate steps ①~③ cyclically until I₁=I₂, and the final insulin infusion amount I₃=I₁=I₂.

33. The closed-loop artificial pancreas insulin infusion control system of claim 30, wherein, the final insulin infusion amount I₃ is optimized by comparing the first insulin infusion amount I₁ and the second insulin infusion amount I₂ with the current statistical analysis result I₄: ${I}_{3} = { \begin{matrix}{I}_{1} , & \left|{I}_{1}-{I}_{4}\right| \leq \left|{I}_{2}-{I}_{4}\right| \\ {I}_{2} , & \left|{I}_{1}-{I}_{4}\right| > \left|{I}_{2}-{I}_{4}\right|\end{matrix}$
